# FASCICULE DE BREVET EUROPEEN

(11) **EP 1 893 555 B1**
(45) Date de publication et mention de la délivrance du brevet: **29.10.2014**
(21) Numéro de dépôt: 06778610.3
(22) Date de dépôt: 16.06.2006
(51) Int. Cl.: C07C 39/205, C07D 311/28, C07D 311/72, C07D 339/04

(54) **BIOPRECURSEUR A BASE DE POLYPHENOL**
BIO-VORSTUFE AUF POLYPHENOLBASIS
POLYPHENOL-BASED BIOPRECURSOR

(30) Priorité: 17.06.2005 FR 0506169
(43) Date de publication de la demande: 05.03.2008
(73) Titulaire: RHODIA CHIMIE, 93300 Aubervilliers (FR); Chanel Parfums Beauté, 92200 Neuilly-sur-Seine (FR)
(72) Inventeur: DELAIRE, Sabine, F-92500 Rueil Malmaison (FR); ADAO, Adrien, F-06650 Opio (FR); DESMURS, Jean-Roger, 06400 CANNES (FR); GELO-PUJIC, Mirjana, F-69360 Serezin Du Rhone (FR); SAINT-JALMES, Laurent, F-69390 Vourles (FR); KASSEM, Tarek, F-34070 Montpellier (FR)
(74) Mandataire: Colombet, Alain André
(86) Numéro de dépôt international: PCT/FR2006/001375
(87) Numéro de publication internationale: WO 2006/134282

(56) Documents cités:
- EP-A- 0 487 404
- EP-A- 0 710 478
- EP-A- 1 205 475
- SAITO A AND AL: "Systematic synthesis of galloyl-substituted procyanidin B1 and B2, and their ability of DPPH radical scavenging activity and inhibitory activity of DNA polymerases" BIOORGANIC & MEDICINAL CHEMISTRY, vol. 13, no. 8, 15 avril 2005 (2005-04-15), pages 2759-2771, XP002368626
- KAMARA B.I. ET AL: "Phenolic metabolites from Honeybush Tea (Cyclopia subternata" JOURNAL OF AGRICALTURAL AND FOOD CHEMISTRY, vol. 52, no. 17, 2004, pages 5391-5395, XP002368627

## Description

L'invention est relative à des bioprécurseurs de molécules biologiquement actives à usage cosmétique ou thérapeutique. Elle vise notamment des utilisations desdits bioprécurseurs de molécules à usage cosmétique ou dermatologique. Elle est aussi relative aux compositions contenant de tels précurseurs et aux méthodes de traitement associées.

L'offre de compositions cosmétiques et/ou dermatologiques est très étendue. Parmi les principes actifs les plus employés dans ces compositions, on peut citer les vitamines, comme les vitamines A, B, C, D, E et F employées pour leurs propriétés contre les surcharges pondérales, le vieillissement de la peau, son dessèchement, sa pigmentation, l'acné et certaines maladies de la peau telles que le psoriasis ou encore pour favoriser la cicatrisation ou la restructuration de la peau.

Les antioxydants sont largement utilisés dans les produits de soins. Les plus utilisés sont les tocophérols et tocotriénols qui représentent une famille homogène de produits constitués d'un reste hydroquinone substitué par un ou plusieurs groupes méthyles et d'une chaîne polyisoprénique plus ou moins saturée. Les plus utilisés sont l'α-tocophérol, le β-tocophérol, le γ-tocophérol, l'α-tocotriénol. Le DL-α-tocophérol, produit de synthèse, est la forme habituelle de la vitamine E dans les spécialités topiques.

Parmi les autres antioxydants couramment utilisés, on peut citer des polyphénols tels que le resvératrol, la quercétine, la lutéoline, les gallates, certaines huiles essentielles, le palmitate d'ascorbyle, les antioxydants phénoliques de synthèse tels que le butylhydroxytoluène (BHT) et le butylhydroxyanisole (BHA).

La grande réactivité chimique de ces antioxydants, en particulier au niveau de leurs groupements phénoliques, les rend sensibles à l'oxydation, ce qui pose des problèmes d'instabilité et de conservation des compositions tant sur le plan de l'activité antioxydante que de l'aspect des formulations, par exemple leur coloration ou encore leur odeur. Il est ainsi connu de protéger les groupements oxydants par des groupes protecteurs qui stabilisent la molécule active lors du stockage et qui sont susceptibles d'être hydrolysés au contact des enzymes de la peau. Bien que les connaissances à ce sujet soient encore limitées, on considère généralement que des lipases, phosphatases, glucosidases, glucocérebrosidases et une sphingomyélinase sont présentes dans le *Stratum corneum* et que des estérases sont présentes dans le *Stratum granulosum* de l'épiderme (U.K. Jain et coll, Proceed. Intern. Symp. Control. Rel. Bioact. Mater., 22, (1995), Controlled Release Society, Inc., p 702-703).

Ainsi, EP-A-0 487 404 divulgue l'utilisation d'un dérivé glucosylé de l'acide ascorbique, dans des compositions dermatologiques. Ce dérivé est hydrolysé par les enzymes cutanées et libère l'acide ascorbique lorsque ces compositions sont appliquées sur la peau. Mais l'utilisation de tels dérivés ne permet pas une libération rapide et en quantité suffisante d'acide ascorbique à la surface de la peau.

EP-A-0 710 478 divulgue un produit pour application topique contenant un précurseur d'un actif cosmétique ou dermatologique (e.g. vitamines telles que rétinol, acide ascorbique) et une lipase. Le précurseur est un ester comportant au moins une fonction ester à chaîne linéaire ou ramifiée, saturée ou insaturée, ayant de 2 à 25, de préférence de 12 à 18 atomes de carbone.

Dans US 2004/0202624, les auteurs proposent des conjugués agissant à la fois comme antioxydants et comme agents protégeant des rayonnements UV. Un flavonoïde sert de molécule de base sur laquelle il est greffé une ou plusieurs molécules possédant des propriétés d'absorption des rayonnements UV. Plus précisément, le flavonoïde porte des radicaux R¹ à R⁵ qui sont choisis parmi -H, -OH et -OA, A désignant l'agent anti-UV. Ce document utilise les propriétés de protection contre les UVA des flavonoïdes pour proposer un conjugué ayant un spectre de protection élargi contre les UVA/B. L'enseignement de ce document englobe de nombreuses possibilités, tant sur le nombre et la nature des molécules anti-UV à greffer, sur le greffage éventuel de radicaux variés favorisant la solubilité dans l'eau ou dans l'huile, que sur le mode d'action et d'administration des conjugués, entre des conjugués ayant une lipophilie suffisante pour pénétrer dans les couches profondes de la peau, l'association à des agents de transport tels que des liposomes, et le transport systémique à partir d'une administration orale. Certains des groupes -OH restant libres sur le conjugué peuvent éventuellement être estérifiés avec un acide carboxylique choisi parmi les acides 2-éthylhexanecarboxylique, butyrique, valérique, hexanoïque, ascorbique et laurique pour favoriser la solubilité dans l'huile, le radical ester pouvant être hydrolysé dans la cellule sous l'effet des estérases. En revanche, ce document ne s'intéresse pas au comportement de ses différents conjugués *in situ* vis-à-vis des enzymes de la peau. Enfin, les conjugués auraient une activité antioxydante liée au flavonoïde permettant de stabiliser les formulations les contenant. Il est supposé que cette activité antioxydante est liée à la présence de groupes -OH libres.

Aucun de ces enseignements ne résout le problème de disposer d'un composé biologiquement actif stable au stockage, ayant néanmoins un effet antioxydant et/ou un autre effet biologique à la surface de la peau et un effet antioxydant et/ou un autre effet biologique, e.g. cosmétique ou dermatologique, dans les couches inférieures, dans des conditions de durabilité et d'efficacité acceptables.

Une autre difficulté majeure, qui n'est pas résolue par l'enseignement antérieur, est d'assurer en même temps une bonne pénétration tissulaire si l'on souhaite délivrer une ou plusieurs molécules actives, par exemple dans l'épiderme, le derme et/ou l'hypoderme, et un pouvoir anti-oxydant efficace dans les différentes couches de la peau, y compris en surface et dans le *Stratum corneum,* en particulier, et donc une activité antioxydante immédiate ou quasi-immédiate à l'application sur la peau.

Il serait par conséquent d'un intérêt majeur de pouvoir disposer de compositions qui soient stables au stockage et qui puissent, après application sur la peau, développer une activité durable ayant un volet anti-oxydant performant à différents niveaux de la peau, y compris en surface (effet protecteur dès l'application ou peu après l'application) et dans le *Stratum corneum,* apte à pénétrer dans les couches inférieures. Il serait d'un plus grand intérêt d'y combiner une activité biologique d'une autre nature, par exemple au niveau du *Sfratum corneum,* du *Stratum granulosum* et/ou des couches inférieures de la peau, à des fins de traitement thérapeutique, en particulier dermatologique, ou cosmétique.

L'invention a donc pour objectif de proposer des molécules et des compositions les comprenant, permettant de remplir ces objectifs.

Ces objectifs sont atteints par un bioprécurseur répondant à la structure :

[A]ₙ - PP - [B]ₘ

dans laquelle :
- PP représente un reste d'un polyphénol où chaque fonction hydroxyle est protégée par un groupement A ou un groupement B ;
- A est une chaîne alkyle substituée ou non, saturée ou insaturée, comprenant de 1 à 20 atomes de carbone, préférentiellement de 1 à 4, qui est liée au polyphénol par :
- une fonction ester carboxylique sur une fonction hydroxyle dudit polyphénol ; ou
- l'intermédiaire d'un espaceur A', dans lequel A est lié à A' par une fonction ester carboxylique, et A' est lié au polyphénol par une fonction ester carboxylique sur une fonction hydroxyle dudit polyphénol ;
- n représente un entier supérieur ou égal à 1, notamment 1, 2, 3, 4 ou 5 ;
- B est un précurseur d'une molécule biologiquement active, qui est lié au polyphénol par :
- une fonction ester carboxylique sur une fonction hydroxyle dudit polyphénol ; ou
- l'intermédiaire d'un espaceur B', dans lequel B est lié à B' par une fonction ester carboxylique, et B' est lié au polyphénol par une fonction ester carboxylique sur une fonction hydroxyle dudit polyphénol ;
- ladite molécule biologiquement active étant choisie parmi : une molécule cosmétiquement active, une molécule pharmaceutiquement active, une molécule dermatologiquement active,
- ladite molécule cosmétiquement active, pharmaceutiquement active ou dermatologiquement active étant choisie parmi : polyphénol, acide lipoïque, vitamines A, B, D, E, F, acides rétinoïques
- m représente un entier supérieur ou égal à 1, notamment 1 ou 2,
le groupement A étant différent du groupement B.

Il doit être compris que lorsque n est supérieur à 1, les groupements protecteurs A peuvent être identiques ou différents. De même, lorsque m est supérieur à 1, les précurseurs B peuvent être identiques ou différents. Le ou les précurseur(s) B qui, comme les groupements A protègent les fonctions hydroxyle du polyphénol, sont différents desdits groupements A en ce qu'il confèrent en outre au bioprécurseur de l'invention une activité thérapeutique, en particulier dermatologique, ou cosmétique.

Le bioprécurseur est susceptible d'être biohydrolysé sous l'action d'enzyme(s) de la peau pour restituer les fonctions hydroxyles, libérer ledit polyphénol et ladite molécule biologiquement active, présente sous forme de son précurseur B dans le bioprécurseur. On dénomme ainsi « bioprécurseur polyfonctionnel » les bioprécurseurs de l'invention comprenant un polyphénol et au moins un précurseur B de molécule biologiquement active.

Il apparaît de manière tout à fait surprenante que le bioprécurseur de l'invention permet la libération contrôlée et progressive de composés biologiquement actifs et notamment dans les différentes couches de la peau. Ceci permet notamment d'améliorer la biodisponibilité des composés biologiquement actifs qui sont véhiculés sous forme d'un composé bioprécurseur. En effet, il apparaît que certaines fonctions hydroxyles de la molécule polyphénol peuvent être libérées alors même que le bioprécurseur se trouve dans les parties supérieures de la peau, telles que le *Stratum corneum,* notamment sous l'action des lipases. Ceci permet au polyphénol de recouvrer son activité anti-oxydante. Le bioprécurseur peut ainsi présenter une activité antioxydante dès sont contact avec la peau et durant toute la traversée de l'épiderme, en particulier du *Stratum corneum.* Les autres fonctions hydroxyles de la molécule polyphénol peuvent aussi être libérées lorsque le bioprécurseur pénètre ensuite dans le tissu vivant, au niveau des parties inférieures ou profondes de la peau, telles que le *Stratum granulosum,* notamment sous l'action des estérases. Ceci permet la libération complète du polyphénol et des molécules biologiquement actives B. Des considérations sur la physiologie de l'absorption cutanée sont mentionnées dans Agache et coli., Encyclopédie Médico-Chirurgicale (Paris) 12-235-C-30, (1995).

De manière avantageuse, les groupements protecteurs du polyphénol sont libérables uniquement lorsque le bioprécurseur de l'invention est placé dans des conditions où les molécules biologiquement actives doivent agir, ce qui permet d'exploiter leurs propriétés de manière optimale. La libération progressive de composés biologiquement actifs du bioprécurseur permet en outre d'éviter des sur-concentrations locales et des effets d'accumulations des molécules actives, qui peuvent provoquer des irritations de la peau.

Le bioprécurseur de l'invention présente notamment une très bonne pénétration cutanée.

On peut aussi utiliser le polyphénol comme vecteur transcutané destiné à libérer un principe actif pharmaceutique et le rendre biodisponible pour des applications autres que dermatologiques. En particulier, le principe actif pharmaceutique libéré est destiné à être ensuite véhiculé par le sang.

Le bioprécurseur de l'invention présente en outre l'avantage d'être parfaitement stable dans une formulation cosmétique, dermatologique ou autrement thérapeutique, et de ne présenter aucun problème de compatibilité avec les excipients et/ou adjuvants généralement utilisés dans lesdites formulations.

Les groupes protecteurs A stabilisent le bioprécurseur lors de son stockage mais sont facilement hydrolysés par les enzymes de la peau et l'hydrolyse commence peu après contact avec celle-ci, ce qui permet au bioprécurseur de développer rapidement les propriétés antioxydantes du polyphénol et les propriétés biologiquement actives de la molécule B. Les groupements A permettent de plus de moduler la cinétique de biohydrolyse du bioprécurseur et donc de libération des composés actifs.

A est avantageusement issu d'un acide carboxylique linéaire, ramifié ou cyclique, par exemple choisi parmi l'acide éthanoïque, l'acide propanoïque, l'acide butanoïque linéaire ou ramifié, l'acide caproïque et l'acide laurique.

Par molécule biologiquement active, on entend des molécules d'origine naturelle, artificielle, synthétique ou issue des biotechnologies, ayant une efficacité biologique. Ainsi, dans le domaine de la cosmétique, la molécule biologiquement active a une efficacité sur la peau via des cibles biologiques en vue d'apporter des effets bénéfiques à la peau, par exemple pour lutter contre le dessèchement, le vieillissement ou la pigmentation de la peau, ou pour favoriser le restructuration de le peau ou son renouvellement cellulaire.

Par précurseur B, on entend un radical susceptible d'être libéré, par hydrolyse enzymatique, sous la forme d'une molécule biologiquement active.

Lorsque l'invention vise une application thérapeutique, dermatologique ou autre, la molécule biologiquement active est un principe actif pharmaceutique, et le précurseur B est choisi en conséquence.

La molécule biologique est choisie parmi : polyphénol, acide lipoïque, vitamines A, B, D, E, F, acides rétinoïques.

On peut particulièrement choisir les molécules biologiquement actives B et le polyphénol afin d'obtenir des effets cosmétiques ou dermatologiques différents, combinés, conjugués ou synergiques.

Lorsque le précurseur B comporte une ou plusieurs fonctions alcool OH, on peut notamment les protéger par un groupe protecteur hydrocarboné, qui peut être du même type que A, par l'intermédiaire d'une fonction ester carboxylique.

Les grandes familles de polyphénols utilisables sont les suivantes :
- Stilbénoïdes : par exemple resvératrol ;
- Flavonoïdes :
   ▪ Les familles des flavonol, flavone, isoflavone, flavanone, anthocyanidines, flavanol, flavilium ;
   ▪ Exemples : quercétine, lutéoline, catéchine, épigallocatéchine ;
- Tannins :
   ▪ Tannins hydrolysables : polyesters des acides gallique et ellagique ;
   ▪ Tannins condensés : proanthocyanidols ;
   ▪ Phlorotannins : e.g. fucofuroeckol ;
- Phénylpropanoïdes : e.g. e.g. curcumine, acide caféique et ses dérivés, et en particulier ses esters, e.g. acide [[(2*E*)-3-(3,4-dihydroxyphényl)-1-oxo-2-propényl]oxy]-3,4-dihydroxybenzènepropanoïque (acide rosmarinique) ;
- Diarylheptanoïdes : curcuminoïdes : par exemple tétrahydrocurcumine ;
- Aurones (e.g. Aureuson) ;
- Alkylpolyphénols (e.g. Cardol) ;
- Dihydrochalcones ;
- Dihydroxycoumarines ;
- Polyhydroxyphénylaminoacides (e.g hydroxytyrosine) ou polyhydroxyphénylamino-alcools (e.g. adrénaline) ;
- Anthracénone (e.g. aloïne) ;
- Benzènediols, benzènetriols (e.g. pyrogallol) ;
- Polyphénols glycosylés : e.g. hespéridine, diosmine ;

On entend, au sens de l'invention, par polyphénol une molécule comprenant au moins un cycle aromatique de type benzène, comprenant éventuellement un ou plusieurs hétéroatomes, et au moins 2 fonctions alcool OH. Les cycles aromatiques peuvent comprendre aucune, une ou plusieurs fonctions alcool OH. Suivant une caractéristique de l'invention, le polyphénol est du type comportant au moins 2 noyaux phénols.

Ainsi, il peut par exemple répondre à la formule suivante : 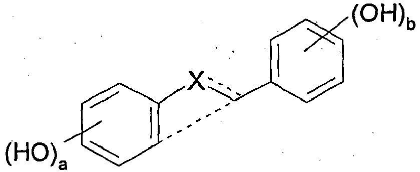 dans laquelle :
- aest1,2,3,4ou5;
- best1,2,3,4ou5;
- X est N, S, O, CH, CH₂, CO, NH ;
- ----représente une double ou une simple liaison ;
- ---- représente une chaîne, éventuellement présente, formant un cycle à 5 ou 6 chaînons, incluant X et comportant une ou plusieurs doubles liaisons, éventuellement un ou plusieurs substituants OH et/ou un ou plusieurs hétéroatomes choisis parmi N, S, O, situés dans le cycle et/ou en substituant.

Le polyphénol peut notamment aussi répondre à la formule suivante : 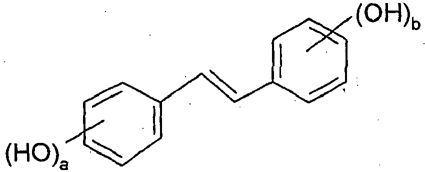 dans laquelle a est 1, 2, 3, 4 ou 5 et b est 1, 2, 3, 4 ou 5, et de préférence, a est 2 et b est 1.

Le polyphénol peut aussi répondre à la formule suivante: 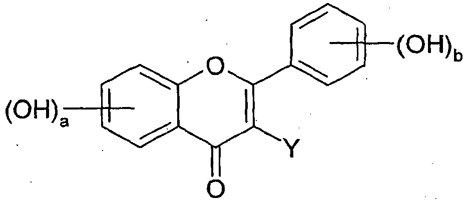 dans laquelle Y est H ou OH, a est 1, 2, 3, ou 4 et b est 1, 2, 3, 4 ou 5, et de préférence, a est 2 et b est 2.

Des exemples préférés de polyphénols sont : resvératrol, lutéoline, quercétine, hydroquinone, pyrocatéchol, acide gallique, hydroxytyrosol, tétrahydrocurcumine, silylmarine, acide ellagique.

Lorsqu'ils sont présents, les espaceurs A' et B', peuvent être, indépendamment l'un de l'autre, une chaîne hydrocarbonée de préférence aliphatique comprenant au moins deux fonctions acides (notamment type diacide) ou au moins une fonction acide et au moins une fonction hydroxyle (notamment type hydroxy-acide) comprenant de 2 à 13 atomes de carbone, de préférence de 2 à 5. Ces espaceurs peuvent également porter d'autres fonctions hydroxyles, acides ou amines. On entend notamment par espaceur, une molécule permettant de lier, par des fonctions esters, deux molécules ayant des fonctions hydroxyles. L'espaceur peut également présenter un effet biologique additionnel, tel que par exemple un effet hydratant pour un hydroxy-acide.

De préférence, A' et B' sont indépendamment l'un de l'autre un reste d'acide succinique, d'acide adipique, d'acide brassylique, d'acide lactique, d'acide salicylique, d'acide 4-hydroxybenzoïque, d'acide férulique, d'acide tartrique, d'acide 2-hydroxybutanoïque, d'acide 3-hydroxybutanoïque ou d'acide 4-hydroxybutanoïque.

La présente invention a en particulier pour objet l'un des bioprécurseurs suivants :
- pentanoate de (*E*)-4-(3,5-diacétoxystyryl)phényl-5-(1,2-dithiolan-3-yle) (ou 3,5-diacétate-4'-lipoate de resvératrol = Res(Ac)₂-Lipoate) ;
- succinate de (*E*)-4-(3,5-diacétoxystyryl)phényl-2,5,7,8-tétraméthyl-2-(4,8,12-triméthyltridécyl)-3,4-dihydro-2*H*-chromen-6-yle (ou 3,5-diacétate-4'-succinyltocophéryle de resvératrol = Res(Ac) ₂-succinate-Vit E) ;
- succinate de 4-[(*E*)-3,5-diacétoxystyryl]phényl-(2*Z*,4*E*,6*Z*,8*E*)-3,7-diméthyl-9-(2,6,6-triméthylcyclohex-1-ényl)nona-2,4,6,8-tetraényle (ou 3,5-diacétate-4'-succinylrétinyle de resvératrol = Res(Ac)₂-succinate-Vit A) ;
- (2*E*,4*E*,6*E*, 8*E*, 10*E*, 12*E*)-4-[(*E*)-3,5-diacétoxystyryl]phényldocosa-2,4,6,8, 10, 12-hexaénoate (ou 3,5-diacétate-4'-docosahexanoate de resvératrol, dérivé de mono-oméga-3) ;
- (*E*)-4-(3,5-diacétoxystyryl)phényl-4-hydroxy-3-méthoxybenzoate (ou 3,5-diacétate-4'-vanillate de resvératrol) ;
- (*E*)-4-(3,5-diacétoxystyryl)phényl-3,4-diacétoxybenzoate (ou 3,5-diacétate-4'-(3,4-diacétoxy)benzoate de resvératrol) ;
- (*E*)-4-[(*E*)-3,5-diacétoxystyryl]phényl-3-(4-hydroxy-3-méthoxyphényl)acrylate (ou 3,5-diacétate-4'-férulate de resvératrol) ;
- (*E*)-4-[(*E*)-3,5-diacétoxystyryl]phényl-3-(4-acétoxy-3-méthoxyphényl)acrylate (ou 3,5-diacétate-4'-acetylférulate de resvératrol) ;
- (*E*)-4-[(*E*)-3,5-diacétoxystyryl]phényl-3-(4-méthoxyphényl)acrylate (ou 3,5-diacétate-4'-(4-méthoxy)cinnamate de resvératrol) ;
- (*E*)-4-(3,5-diacétoxystyryl)phényl-2-acétoxybenzoate (ou 3,5-diacétate-4'-acétylsalycilate de resvératrol) ;
- 7-acétoxy-2-(3,4-diacétoxyphényl)-4-oxo-4*H*-chromen-5-yl-5-(1,2-dithiolan-3-yl)pentanoate (ou triacétate-lipoate de lutéoline) ;
- 7-acétoxy-2-(3,4-diacétoxyphényl)-4-oxo-4*H*-chromén-5-yl-2,5,7,8-tétraméthyl-2-(4,8,12-triméthyltridécyl)-3,4-dihydro-2*H*-chromén-6-ylsuccinate (ou triacétate-monosuccinyltocophéryle de lutéoline) ;
- 2-méthoxy-4-(7-(3-méthoxy-4-(prop-1-én-2-yloxy)phényl)-3,5-dioxoheptyl)phényl-5-(1,2-dithiolan-3-yl)pentanoate (ou monoacétate-monolipoate de tétrahydrocurcumine) ;
- 2-méthoxy-4-(7-(3-méthoxy-4-(prop-1-én-2-yloxy)phényl)-3,5-dioxoheptyl)phényl-2,5,7,8-tétraméthyl-2-(4,8,12-triméthyltridécyl)-3,4-dihydro-2*H*-chromén-6-ylsuccinate (ou monoacétate-monosuccinyltocophéryl de tétrahydrocurcumine) ;

Parmi les bioprécurseurs définis ci-dessus, on préfère particulièrement le pentanoate de (*E*)-4-(3,5-diacétoxystyryl)phényl-5-(1,2-dithiolan-3-yle) (ou 3,5-diacétate-4'-lipoate de resvératrol = Res(Ac)₂-Lipoate) et le succinate de (*E*)-4-(3,5-diacétoxystyryl)phényl-2,5,7,8-tétraméthyl-2-(4,8,12-triméthyltridécyl)-3,4-dihydro-2*H-*chromen-6-yle (ou 3,5-diacétate-4'-succinyltocophéryle de resvératrol = Res(Ac)₂-succinate-Vit E).

D'une manière générale, l'invention concerne l'application des bioprécurseurs décrits précédemment dans le domaine thérapeutique, par exemple dermatologique, ou cosmétique.

La présente invention a également pour objet les compositions contenant au moins un bioprécurseur selon l'invention, dans une formulation topique acceptable pour l'application envisagée, thérapeutique, dermatologique ou cosmétique. L'invention concerne aussi l'utilisation d'un bioprécurseur tel que défini précédemment pour la préparation d'une composition thérapeutique, dermatologique ou cosmétique.

À titre d'exemples, et de manière non limitative, les compositions contenant au moins un bioprécurseur selon l'invention peuvent être des compositions de soin pour le visage ou pour le corps, des compositions de soin amincissant, des compositions solaires, des compositions parfumées, des compositions de maquillage, telles que des fonds de teint ou des poudres, des compositions pour les lèvres sous forme de baumes, de « lipsticks » ou de « gloss », ou des compositions de mascara, des compositions d'hygiène corporelle, telles que des gels douche ou des déodorants.

Le milieu acceptable comprend généralement de l'eau, et/ou un mélange d'eau et de corps gras, et/ou un mélange de corps gras, et/ou un mélange d'eau et de silicone. Le domaine technique offre un large choix de types de formulations topiques, et l'on peut citer, sans vouloir être exhaustif : émulsions (e.g. H/E, E/H, E/H/E, H/E/H, E/Si ; E = eau, H = huile, Si = silicone), suspension, solution, pâte, pommade, gel aqueux, gel hydro-alcoolique, crème, lotion, poudre, savon, spray, mousse.

Ces compositions peuvent contenir également des additifs cosmétiques ou dermatologiques acceptables. Ces additifs peuvent être, en particulier mais non limitativement, des tensioactifs, des corps gras, tels que des huiles, des molécules actives libres, telles que des hydratants ou des actifs hydrophiles ou lipophiles, des conservateurs, des parfums, des chélateurs, des pigments, des filtres, des séquestrants, des matières colorantes, des charges, des humectants, des épaississants, tels que des gélifiants, des agents de texture, des agents de saveurs, des solvants, etc.

De manière non limitative, des additifs qui peuvent être employés dans les compositions de la présente invention sont les suivants :
- les huiles, choisies notamment parmi : les huiles de silicone, linéaires ou cycliques, volatiles ou non volatiles, telles que les polydiméthylsiloxanes (diméthicones), les polyalkylcyclosiloxanes (cyclométhicones) et les polyalkylphénylsiloxanes (phényldiméthicones) ; les huiles synthétiques telles que les huiles fluorées, les alkylbenzoates et les hydrocarbures ramifiés tels que le polyisobutylène ; les huiles végétales et notamment de soja ou de jojoba ; et les huiles minérales telles que l'huile de paraffine ;
- les cires, telles que l'ozokérite, la cire de polyéthylène, la cire d'abeille ou la cire de carnauba ;
- les élastomères de silicone obtenus notamment par réaction, en présence d'un catalyseur, d'un polysiloxane ayant au moins un groupe réactif (hydrogène ou vinyle, notamment) et portant au moins un groupe alkyle (notamment méthyle) ou phényle, terminal et/ou latéral, avec un organosilicone tel qu'un organohydrogénopolysiloxane ;
- les tensioactifs, de préférence émulsionnants, qu'ils soient non ioniques, anioniques, cationiques ou amphotères, et en particulier les esters d'acides gras et de polyols tel que les esters d'acides gras et de glycérol, les esters d'acides gras et de sorbitan, les esters d'acides gras et de polyéthylèneglycol et les esters d'acides gras et de sucrose ; les éthers d'alcools gras et de polyéthylèneglycol ; les alkylpolyglucosides ; les polysiloxanes modifiés polyéthers ; la bétaïne et ses dérivés ; les polyquaterniums ; les sels de sulfate d'alcools gras éthoxylés ; les sulfosuccinates ; les sarcosinates ; les alkyl- et dialkyl-phosphates et leurs sels ; et les savons d'acides gras ;
- les co-tensioactifs tels que les alcools gras linéaires et en particulier les alcools cétylique et stéarylique ;
- les épaississants et/ou gélifiants, et en particulier les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique ; la gomme de xanthane ou de guar ; les dérivés cellulosiques ; et les gommes de silicone (diméthiconol) ;
- les humectants, tels que les polyols, dont la glycérine, le propylène glycol et les sucres, et les glycosaminoglycanes tels que l'acide hyaluronique et ses sels et esters ;
- les filtres organiques, tels que les dérivés de dibenzoylméthane (dont le butyl-méthoxydibenzoylméthane), les dérivés d'acide cinnamique (dont l'éthylhexyl-méthoxycinnamate), les salicylates, les acides para-aminobenzoïques, les β-β'-diphénylacrylates, les benzophénones, les dérivés de benzylidène-camphre, les phénylbenzimidazoles, les triazines, les phénylbenzotriazoles et les dérivés anthraniliques ;
- les filtres inorganiques, à base d'oxydes minéraux sous forme de pigments ou de nanopigments, enrobés ou non, et en particulier à base de dioxyde de titane ou d'oxyde de zinc ;
- les colorants;
- les conservateurs ;
- les charges, et en particulier les poudres à effet « soft-focus », qui peuvent être notamment choisies parmi les polyamides, la silice, le talc, le mica, les fibres (notamment de polyamide ou de cellulose) ;
- les séquestrants tels que les sels d'EDTA ;
- les parfums ;
et leurs mélanges, sans que cette liste ne soit limitative.

Des exemples de tels adjuvants sont cités notamment dans le Dictionnaire CTFA *(*International Cosmetic Ingrédient Dictionary and Handbook, publié par The Cosmetic, Toiletry and Fragrance Association, 9me Édition, 2002).

Bien entendu, l'homme de l'art veillera à choisir ce ou ces éventuels additifs de manière telle que les propriétés avantageuses attachées intrinsèquement à la composition de l'invention ne soient pas, ou substantiellement pas, altérées par la ou les adjonctions envisagées.

Les bioprécurseurs et compositions selon l'invention sont avantageusement insensibles ou peu sensibles aux facteurs extérieurs tels que la lumière ou la chaleur, aux variations de pH et aux additifs tels que des tensioactifs, des solvants, des catalyseurs métalliques. Cette stabilité permet de conserver l'efficacité recherchée et l'aspect visuel et l'odeur des compositions.

Les quantités des différents constituants des compositions sont celles classiquement utilisées dans le domaine considéré, e.g. cosmétique et dermatologique.

La quantité de bioprécurseur dans les compostions de l'invention dépend de nombreux facteurs, parmi lesquels on peut citer, de manière non limitative le type de formulation, la nature du bioprécurseur, le mode d'administration, la sévérité de la pathologie éventuelle à traiter, l'intensité de l'effet souhaité, et autres. La quantité de bioprécurseur peut par exemple être comprise entre 0,001 % et 20%, de préférence entre 0,005% et 15%, de préférence encore entre 0,01% et 10%, avantageusement entre 0,05% et 5%, ou encore entre environ 0,08% et environ 2%.

La présente invention a aussi pour objet l'utilisation d'un polyphénol tel que décrit ici, notamment comprenant tout ou partie de ses fonctions hydroxyle protégées par des groupements A, comme vecteur permettant de faire pénétrer une ou plusieurs molécules biologiquement actives, telles que celles décrites ici, dans la peau, et plus particulièrement dans une ou plusieurs des couches de la peau.

Les bioprécurseurs de l'invention peuvent être fabriqués par différents procédés connus de l'art antérieur, tel que des procédés d'acylation de dérivés de phénols (voir Protective Groups in Organic Synthesis, T.W. Green, second edition, (1991), page 162), des procédés de déprotection sélective d'esters de phénols en utilisant des systèmes enzymatiques.

La présente invention concerne notamment un procédé de fabrication de bioprécurseurs de l'invention comprenant au moins les étapes suivantes :
a) protection du polyphénol par per-estérification, tel que peracétylation, par un composé A-Z, Z étant une fonction susceptible de réagir avec une fonction OH du polyphénol pour générer la liaison ester entre le polyphénol et A,
b) déprotection sélective de manière à obtenir une ou plusieurs fonctions OH libres du polyphénol, et
c) couplage de l'intermédiaire obtenu après l'étape b) avec la molécule biologiquement active B préalablement activée.

Pour ce qui concerne l'étape a), les peracétates peuvent être préparés par peracétylation du resvératrol dans des conditions standards en présence de pyridine et d'un excès d'anhydride acétique comme décrit par H. Aft dans Journal of Organic Chemistry, 26, (1961), 1958-1963. La réaction s'effectue en chauffant à 80°C pendent quelque heures. La synthèse est classique et conduit au produit désiré avec de très bons rendements.

. Pour ce qui concerne la déprotection sélective de l'étape b), la désestérification, e.g. désacétylation, sélective d'un composé perestérifié, e.g. peracétylé, peut être réalisée à l'aide d'une enzyme, comme par exemple une lipase microbienne, comme décrit par Nicolosi et coli. dans Journal of Molecular Catalysis B : Enzymatic, 16, (2002), 223-229. L'enzyme utilisée est par exemple la lipase de *Candida antarctica* immobilisée sur une résine de polypropylène. La lipase est commercialisée par Novo Nordisk sous le nom Novozym SP435^{®}.

La désestérification ou la désacétylation catalysée par la lipase peut être réalisée soit par la réaction d'hydrolyse dans le milieu aqueux, ou bien par une alcoolyse dans un solvant organique. Le plus souvent on utilise un milieu « alcool » et on parle d'une alcoolyse. La réaction s'effectue dans des conditions « douces » de température (15 à 75°C) et de pH (5-8, si milieu aqueux).

Le produit est généralement obtenu par simple filtration de l'enzyme et par concentration sous pression réduite du milieu réactionnel.

À l'étape c), le couplage de l'intermédiaire obtenu après l'étape b) avec la molécule biologiquement active B préalablement activée peut être effectué par une méthode de couplage connue de l'homme du métier (DCC = dicyclocarbodiimide, chloruration d'une fonction acide en chlorure d'acide, etc.).

La chloruration avec le chlorure de thionyle est la méthode la plus utilisée car les produits formés sont facilement obtenus et utilisés sans isolement (voir J. S. Pizey, Synthetic Reagents, 1, (1974), 321). La réaction est réalisée à température ambiante dans un des solvants anhydres tel que dichlorométhane.

Le couplage entre un alcool et un acide peut être catalysé par un agent de déshydratation comme par exemple dicyclohexylcarbodiimide (DCC). Les conditions réactionnelles sont décrites par M. Smith et collaborateurs dans J. Am. Chem. Soc., 80, (1958), 6204.

La présente invention concerne aussi un procédé de traitement cosmétique de la peau consistant à appliquer sur ladite peau une composition telle que décrit précédemment, dont le bioprécurseur contient un précurseur B de composé actif sur le plan cosmétique. L'invention concerne notamment un procédé cosmétique de libération de polyphénol et de molécules actives B au niveau du *Stratum corneum* ou des tissus vivants de la peau par application topique sur la peau d'une composition telle que définie précédemment.

L'invention concerne aussi un procédé de traitement dermatologique consistant à appliquer sur la peau une composition telle que précédemment décrite, dont le bioprécurseur contient un précurseur B de composé actif du point de vue dermatologique.

L'invention concerne aussi un procédé de traitement thérapeutique consistant à appliquer sur la peau une composition telle que décrit précédemment, dont le bioprécurseur contient un précurseur B de composé pharmaceutique à délivrer par voie transcutanée, notamment à action systémique.

L'invention va être maintenant décrite à l'aide de modes de réalisation pris à titre d'exemples non limitatifs.

### Exemple 1 : Synthèse de (E)-4-(3,6-diacétoxystyryl)phényl-5-(1,2-dithiolan-3-yl)pentanoate (3,5-diacétate-4'-lipoate de resvératrol = Res(Ac)₂-Lipoate)

L'ester mixte 3,5-diacétyl-4'-lipoylresvératrol (1) est obtenu en quatre étapes par estérification de diacétate de resvératrol) avec le chlorure de lipoyle en présence de la triéthylamine et du DMAP (diméthylaminopyridine) dans le tétrazhydrofurane (THF) à 0 °C.

### a) Préparation du 3,5,4'-triacétate de resvératrol (Res(Ac)₃)

A une solution de resvératrol (156 g d'une pureté de 96%, 0,66 mole) dans l'anhydride acétique (372 mL, 6 eq) la pyridine (10 eq) est ajoutée goutte à goutte sous agitation à température ambiante. Le milieu réactionnel est chauffé à 80 °C pendant 1 heure. Le produit est obtenu après la précipitation dans 3 L d'eau, filtration et deux lavages successifs à l'eau. Le triacétate de resvératrol est obtenu avec un rendement quantitatif et une pureté HPLC de 99 % (300 nm) et pureté molaire mesurée par RMN du proton de 98 %. Point de fusion = 120-121 °C. RMN ¹H (DMSO / HMDS ; 300 MHz): 2,21 (s, 3H) ; 2,23 (s, 6H) ; 6,85 (t, 2,2Hz, 1H) ; 7,10 (d, 8,5Hz, 2H) ; 7,15 (d, 16,5Hz, 1H) ; 7,23,(d, 2,2Hz, 2H) ; 7,28 (d, 16,5Hz, 1H) ; 7,57 (d, 8,5Hz, 2H).

### b) Synthèse du 3,5-diacétate de resvératrol (Res(Ac)₂)

Le diacétate de resvératrol est obtenu par alcoolyse enzymatique du triacétate de resvératrol (1.1) avec la Novozyme® SP435 comme décrit par Nicolosi et coll. (Journal of Molecular Catalysis B: Enzymatic, 16, (2002), 223-229) avec certaines modifications, a savoir : le tert-butylmethyléther (TBME) a été remplacé par l'acétonitrile et la quantité d'enzyme a été diminuée à 20 % p/p. L'alcoolyse du triacétate de resvératrol est réalisée à l'échelle de 200 g/L du triacétate de resvératrol avec du n-butanol en présence de 20 % p/p de Novozyme SP435 dans l'acétonitrile à 65°C et en 15 heures. Le produit brut est obtenu par filtration de l'enzyme et par concentration sous pression réduite du milieu réactionnel. Le produit brut est un mélange de 88 % du diacétate (1.2) et de 12 % du monoacétate. Il a été purifié par chromatographie sur une colonne de silice avec cyclohexane / acétate d'éthyle (4/1 v/v) comme éluant. Le produit pur est obtenu avec un rendement de 50 % et une pureté molaire mesurée par RMN du proton de 99 %.
Point de fusion = 134-136 °C.
RMN ¹H (DMSO / HMDS ; 300 MHz): 2,22 (s, 6H) ; 6,72 (d, 8,5Hz, 2H) ; 6,78 (t, 2,2Hz, 1 H) ; 6,93 (d, 16,2Hz, 1H) ; 7,15 (d, 16,2Hz,, 1H) ; 7,16 (d, 2,2Hz, 2H) ; 7,36 (d, 8,5Hz, 2H) ; 9,58 (br s, OH).

### c) Synthèse du 3,5-diacétate-4'-lipoate de resvératrol (Res(Ac)₂-Lipoate)

La synthèse du chlorure d'acide lipoïque (acide DL-thioctique) a été réalisée sous argon à température ambiante en suivant un protocole standard : l'acide lipoïque (5 g, 24 mmol) est solubilisé dans du dichlorométhane (40 mL) et du SOCl₂ (1,3 eq) est ajouté goutte à goutte. Après 1 heure d'agitation à température ambiante, le chlorure d'acide lipoïque formé est coulé sur une solution de diacétate de resvératrol (1.2) (6 g, 19 mmol) dans du THF (100 mL), contenant la triéthylamine (3 eq) et le DMAP (0,45 eq). Après 1 heure le mélange réactionnel est dilué avec 100 mL CH₂Cl₂, puis lavé avec deux fois 90 mL de HCl 5 % v/v. La phase organique est séchée sur MgSO₄ anhydre, filtrée, puis concentrée à l'évaporateur rotatif. Le produit brut (11,4 g) est purifié par chromatographie sur une colonne de silice avec cyclohexane / acétate d'éthyle (4/1 v/v) comme éluant. Les fractions contenant le produit pur sont évaporées pour conduire à 5,95 g de 3,5-diacétate-4'-lipoate de resvératrol 1 (49 % de rendement) d'une pureté molaire mesurée par RMN du proton de 96%.
Point de fusion = 79-81 °C.
RMN ¹H (DMSO / HMDS ; 300 MHz): 1,42 (m, 2H) ; 1,60 (m, 4H) ; 1,84 (m, 1H) ; 2,23 (s, 6H) ; 2,36 (m, 1H) ; 2,53 (t, 7,4 Hz, 2H) ; 3,09 (m, 2H) ; 3,54 (m, 1H) ; 6,85 (t, 2,2 Hz, 1H) ; 7,08 (d, 8,5 Hz, 2H) ; 7,15 (d, 16,7 Hz, 1H) ; 7,23 (d, 2,2 Hz, 2H) ; 7,28 (d, 16,7 Hz, 1 H) ; 7,58 (d, 8,5 Hz, 2H).

### Exemple 2 : Synthèse de (E)-4-((E)-3,5-diacétoxystyryl)phényl 3-(4-acétoxy-3-méthoxyphényl)acrylate (3,5-diacétate-4'-acétylférulate de resvératrol)

L'ester mixte 3,5-diacétate-4'-acétyleférulate de resvératrol est obtenu en cinq étapes par estérification de diacétate de resvératrol avec le chlorure d'acide férulique O-acétyle en présence de la triéthylamine et du DMAP dans THF à 0°C.

### a) Synthèse de l'acide (E)-3-(4-acétoxy-3-méthoxyphényl)acrylique (acide férulique O-acétyle)

L'acide férulique (10 g, 51 mmol) est solubilisé dans 100 mL de THF anhydre, sous argon. On charge rapidement la triéthylamine (8,7 mL, 61 mmol, 1,2 eq), puis la DMAP (3,34 g, 27 mmol, 0,5 eq) à température ambiante et sous argon. On charge enfin l'anhydride acétique (5,9 mL, 61 mmol, 1,2 eq) en 3 min, à température ambiante. On maintient sous agitation, à température ambiante, pendant 18 h, puis on transfère le milieu dans une ampoule à décanter. On dilue ce milieu avec 100 mL de THF, on acidifie avec 40 mL de HCl 5% v/v et on lave la phase organique avec 5 x 40 mL d'H₂O. La phase organique finale est séchée sur MgSO₄, filtrée, puis évaporée à sec à l'évaporateur rotatif (40°C, 30mbar). On obtient 9,5 g d'un solide brut jaune avec un rendement brut de 79 % et une pureté molaire dosée par RMN de 86 %. Le produit est engagé dans l'étape suivante sans purification.

### b) Acétate de (E)-4-(3-chloro-3-oxoprop-1-ényl)-2-méthoxyphényle (chlorure de l'acide férulique O-acétyle)

À une suspension de l'acide férulique O-acétyle (6,24 g, 26 mmol) dans 30 mL de chloroforme, on charge le DMF (200 µL, 26 mmol), puis le chlorure de thionyle (2,35 mL, 32 mmol, 1,2 eq), à température ambiante. On chauffe à reflux pendant 4 h, puis on laisse le milieu revenir à température ambiante et on évapore à sec le milieu à l'évaporateur rotatif. On obtient un solide jaune avec un rendement de brut quantitatif. Le produit est engagé dans l'étape suivante sans purification.

### c) Acrylate de (E)-4-[(E)-3,5-diacétoxystyryl]phényl 3-(4-acétoxy-3-méthoxyphényl)-acrylate (3,5-diacétate-4'-acetylférulate de resvératrol)

On charge le diacétate de resvératrol (1,92 g, 5 mmol) qu'on solubilise dans 10 mL de THF anhydre. On charge rapidement la triéthylamine (2,15 mL, 15 mmol, 3 eq), puis la DMAP (121 mg, 1 mmol, 0,2 eq). On charge à température ambiante une solution de chlorure de l'acide férulique O-acétyle (1,27 g, 5 mmol, 1 eq) solubilisé dans 10 mL de THF anhydre + 3 mL de dichlorométhane. Après 4 h d'agitation à température ambiante, le milieu est transféré dans une ampoule à décanter, dilué avec 50 mL de dichlorométhane et acidifié avec HCl 5% v/v. La phase organique est lavée avec 20 mL de NaHCO₃ aqueux saturé, puis avec 4 x 30 mL d'H₂O. La phase organique finale est séchée sur MgSO₄ filtrée et évaporée à sec à l'évaporateur rotatif.

On obtient 2,84 g de solide brut avec un rendement quantitatif. Le produit brut est solubilisé dans du dichlorométhane, puis précipité avec du pentane. Le produit final est isolé avec un rendement de 50 % et une pureté molaire de 76 % et 24 % de diacétate de resvératrol de départ.
¹H (CDCl₃, HMDS, 300 MHz): 2,22 (s, 9H) ; 3,79 (s, 3H) ; 6,50 (d, 15,9 Hz, 1H) ; 7,09 (d, 8,5 Hz, 2H) ; 7,43 (d, 8,5 Hz, 2H) ; 7,75 (d, 15,9 Hz, 2H).

### Exemple 3 : Synthèse de succinate de (E)-4-(3,5-diacétoxystyryl)phényl-2,5,7,8-tétraméthyl-2-(4,8,12-triméthyltridécyl)-3,4-dihydro-2H-chromén-6-yle (3,5-diacétate-4'-monosuccinyltocophéryle de resvératrol = Res(Ac)₂-succinyl-Vit E)

L'ester mixte 3,5-diacétate-4'-succinyltocophérol est obtenu en trois étapes par estérification de diacétate de resvératrol avec le chlorure de tocophérylsuccinate en présence de triéthylamine et du DMAP dans THF à 0°C.

### a) Synthèse de l'acide 4-oxo-4-(2,5,7,8-tétraméthyl-2-(4,8,12-triméthyltridécyl)-3,4-dihydro-2H-chromen-6-yloxy)butanoïque (vitamine E succinate)

L'α-tocophérol racémique (10,2 g, 23 mmol) et l'anhydride succinique (1,5 eq) sont dissous dans 50 mL de dichlorométhane. La DMAP (0,5 eq) et la triéthylamine (1,05 eq) y sont rajoutés et la réaction est suivie par chromatographie en couche mince (CCM) (acétate d'éthyle/cyclohexane 50/50 v/v). Le mélange réactionnel est agité pendant 1 nuit à température ambiante et à l'abri de la lumière. Le mélange est dilué avec 40 mL de dichlorométhane, lavé avec HCl 5 % v/v, puis avec H₂O et la phase organique est séchée sur MgSO₄, puis évaporée à sec à l'évaporateur rotatif. Le produit brut est obtenu avec un rendement quantitatif. Ce produit brut est solubilisé dans de l'éther diéthylique et cette solution est filtrée à travers un cake de silice. Le filtrat obtenu est évaporé à sec à l'évaporateur rotatif. On obtient une huile qui se solidifie à 4°C. Le produit est isolé avec un rendement de 70 % et une pureté molaire de 94 % (mesuré par RMN ¹³C).
¹³C (CDCl₃, TMS, 75 MHz): 11,7 (CH₃Ph) ; 11,9 (CH₃Ph) ; 12,8 (CH₃Ph) ; 19,6 (2CH₃ chaîne) ; 20,5 (CH₂ cycle) ; 21,0 (CH₂ chaîne) ; 22,5 (CH₃ chaîne) ; 22,6 (CH₃ chaîne) 23,8 (CH₃ cycle) ; 24,4 (CH₂ chaîne) ; 24,7 (CH₂ chaîne) ; 27,9 (CH chaîne) ; 28,5 (CH₂C=O) ; 28,9 (CH₂C=O) ; 31,0 (CH₂ cycle) ; 32,7 (CH chaîne) ; 32,7 (CH chaîne) ; 37,5 (4 CH₂ chaîne) ; 39,3 (CH₂ chaîne) ; 39,9 (CH₂ chaîne) ; 75,0 ; Q cycle) ; 117,3 (Q arom) ; 123,0 (Q arom) ; 124,8 (Q arom) ; 126.7 (Q,arom) ; 140,4 (Q arom) ; 149,4 (Q arom) ; 170,7 (COOPh) ; 177,8 (COOH).

### b) Synthèse du 2,5,7,8-tétraméthyl-2-(4,8,12-triméthyltridécyl)-3,4-dihydro-2H-chromén-6-yl-4-chloro-4-oxobutanoate (chlorure de vitamine E succinate)

La synthèse du chlorure de tocophérylsuccinate a été réalisée sous argon à température ambiante en suivant un protocole standard (un léger excès de SOCl₂, triéthylamine, dichlorométhane) à température ambiante et sous argon. Le chlorure d'acide n'est pas isolé et est engagé tel quel dans l'étape suivante d'estérification.

### c) Synthèse de succinate de (E)-4-(3,5-diacétoxystyryl)phényl-2,5,7,8-tétraméthyl-2-(4,8,12-triméthyltridécyl)-3,4-dihydro-2H-chromén-6-yle (3,5-diacétate-4'-succinyltocophéryle de resvératrol = Res(Ac)₂-succinate-Vit E)

La solution de 3,5-diacétate de resvératrol (1.2) (2,21 g), triéthylamine (1,1 eq) et DMAP (0,2 eq) dans le THF (10 ml) est coulée directement dans la solution de chlorure de tocophérylsuccinate dans CH₂Cl₂ obtenue précédemment à l'étape b).

On obtient un rendement quantitatif du produit brut. Après la purification sur une colonne de silice avec un mélange acétate d'éthyle/cyclohexane 30/70 v/v comme éluant, on obtient un rendement de 55 % de diacétate de resvératrol-succinate-Vit E d'une pureté molaire de 98 % analysée par RMN.
RMN ¹H (CDCl₃ / HMDS ; 300 MHz): 0,80 (m, 12H) ; 1,04 - 1,49 (m, 24H) ; 1,72 (m, 2H) ; 1,92 (s, 3H) ; 1,96 (s, 3H) ; 2,03 (s, 3H) ; 2,24 (s, 6H) ; 2,53 (t, 6,6Hz, 2H) ; 2,97 (m, 4H) ; 6,77 (t, 2,2Hz, 1 H) ; 6,89 (d, 16,2Hz, 1H) ; 6,99 (d, 16,2Hz, 1 H) ; 7,03 (d, 8,5Hz, 2H) ; 7,05 (d, 2,2Hz, 2H) ; 7.41 (d, 8,5Hz, 2H).
RMN ¹³C (CDCl₃ / TMS ; 75 MHz): 11,7 (CH₃Ph) ; 12,0 (CH₃Ph) ; 12,9 (CH₃Ph) ; 19,6 (2CH₃ chaîne) ; 20,5 (CH₂ cycle) ; 21,0 (CH₂ chaîne) ; 21,0 (2CH₃C=O) ; 22,6 (CH₃ chaîne) ; 22,6 (CH₃ chaîne) ; 23,9 (CH₃ cycle) ; 24,4 (CH2 chaîne) ; 24,7 (CH₂ chaîne) ; ((2,.8 CH₂ cyclohexane)) ; 27,9 (CH chaîne) ; 28,7 (CH₂C=O) ; 29,2 (CH₂C=O) ; 31,0 (CH₂ cycle) ; 32,6 (CH chaîne) ; 32,7 (CH chaîne) ; 37,5 (4CH₂ chaîne) ; 39,3 (CH₂ chaîne) ; 39,9 (CH₂ chaîne) ; 75,0 (Q cycle) ; 114,3 (CH arom) ; 116,8 (2CH arom) ; 117,3 (Q arom) ; 121,8 (2CH arom) ; 123,0 (Q arom) ; 124,9 (Q arom) ; 126;6 (Q arom) ; 127,1 (CH éthylénique) ; 127,6 (CH arom) ; 129,6 (CH éthylénique) ; 134,5 (Q arom) ; 139,5 (Q arom) ; 140,4 (Q arom) ; 149,4 (Q arom) ; 150,3 (Q arom) ; 151,2 (Q arom) ; 168,9 (2COCH₃) ; 170,7 (COOPh) ; 170,7 (COOPh).

### Exemple 4: Synthèse de 7-acétoxy-2-(3,4-diacétoxyphényl)-4-oxo-4H-chromen-5-yl 2,5,7,8-tétraméthyl-2-(4,8,12-triméthyltridécyl)-3,4-dihydro-2H-chromen-6-yl succinate (triacétate-succinyltocopheryl de lutéoline = Lut(Ac)₃-succinate-Vit E)

L'ester mixte de lutéoline et de vitamine E (triacétate-monosuccinyltocophéryl de lutéoline) est obtenu en quatre étapes par estérification de triacétate de lutéoline avec le chlorure de vitamine E succinate en présence de triéthylamine et du DMAP dans THF à 0 °C.

### a) Synthèse du 3',4',7-triacétate de lutéoline (Lut(Ac)₃)

Sur une suspension de lutéoline (40,8 g, 0,14 mole) dans le tert-butylméthyléther (1 L, 60 eq), on coule rapidement de la triéthylamine (3,5 eq, 68 mL), puis lentement sous argon et à température ambiante de l'anhydride acétique (3,1 eq, 41 mL). Le milieu est ensuite chauffé à 50°C pendant 2 heures. Après la filtration du milieu réactionnel, le produit obtenu est repris dans le dichlorométhane, puis lavé deux fois par une solution HCl à 5 % v/v. La phase organique est séchée sur sulfate de magnésium. Après évaporation du solvant, le triacétate de lutéoline est obtenu avec un rendement de 80 % et une pureté molaire mesurée par RMN du proton de 85,5 %.
¹H (CDCl₃, HMDS, 300 MHz): 2,25 (s, 6H) ; 2,27 (s, 3H) ; 6,51 (d, 2,2 Hz, 1 H) ; 6,60 (s, 1H) ; 6,78 (d, 2,2 Hz, 1H) ; 7,30 (d, 8,2 Hz, 1H) ; 7,66 (m, 1H) ; 7,68 (dd, 8,2 et 2,2 Hz, 1 H) ; 12,53 (s).
¹³C (CDCl₃, TMS, 75 MHz): 20,5 (CH₃CO) ; 20,6 (CH₃CO) ; 21,1 (CH₃CO) ; 100,9 (CH arom) ; 105,5 (CH arom), 106,4 (CHCO) ; 108,7 (Q arom) ; 121,8 (CH arom) ; 124,3 (CH arom) ; 124,6 (CH arom) ; 129,4 (Q arom) ; 142,6 (Q arom) ; 145,1 (Q arom) ; 156,0 (Q arom) ; 156,5 (Q arom) ; 161,8 (Q arom) ; 162,7 (Q arom) ; 167,6 (COAc) ; 167,8 (COAc) ; 168,2 (COAc).

### b) Synthèse de triacétate-succinyltocophéryle de lutéoline (Lut(Ac)₃-succinate-Vit E)

Dans une suspension de triacétate de lutéoline (2,28 g, 5,5 mmol) dans 50 mL de dichlorométhane, la triéthylamine (0,75 mL, 1,1 eq) et la 4-DMAP (120 mg, 0,2 eq) sont additionnées. On coule ensuite lentement sous argon et à température ambiante 1 équivalent de la solution du chlorure d'α-tocophérylsuccinate obtenue comme décrit précédemment. Après 17 heures de réaction, la suspension obtenue est reprise dans le dichlorométhane, puis lavée deux fois par une solution HCl à 5 % v/v. La phase organique est alors séchée sur sulfate de magnésium. Après évaporation du solvant, on obtient 3,4 g, soit un rendement de 52 %.
¹H (CDCl₃, HMDS, 300 MHz): 0,80 (m, 12H) ; 1,04 - 1,5 (m, 24H) ; 1,72 (m, 2H) ; 1,90 (s, 3H) ; 1,95 (s, 3H) ; 2,01 (s, 3H) ; 2,28 (s, 9H) ; 2,52 (t, 6,8 Hz, 2H) ; 2,96 (m, 4H) ; 6,54 (s, 1H) ; 6,78 (m-car mélange, 1 H) ; 7,28 (m-car mélange, 2H) ; 7,66 (m, 2H).

### Exemple comparatif 5 : Synthèse de 3,5,4'-tricaproate de resvératrol

### (Res(caproate)₃)

Le tricaproate (trihexanoate) de resvératrol est obtenu par estérification de resvératrol avec le chlorure de hexanoyle en présence de triéthylamine dans THF à température ambiante.

À une solution de resvératrol (4 g ; 17,5 mmol) dans du THF (100 mL) la triéthylamine (8 mL ; 3,3 eq) est ajoutée goutte à goutte sous agitation à température ambiante. Le milieu réactionnel est refroidi à 0 °C et de chlorure de hexanoyle (8,2 mL ; 3 eq) est ajouté goutte à goutte. Le milieu réactionnel est agité à température pendant 72 h. Le milieu est ensuite lavé par trois fois 30 mL de solution saturée de bicarbonate (pH 11). La phase aqueuse est extraite avec du dichlorométhane et cette phase organique est séchée sur MgSO₄ anhydre. Après concentration sous pression réduite, on récupère 5,2 g d'une huile jaune d'une pureté massique dosée par RMN de 82 %. Le produit pur est obtenu avec un rendement de 80 % après une purification sur la colonne de silice avec acétate d'éthyle / cyclohexane (20/80 v/v) comme éluant.
¹H (DMSO, HMDS, 300 MHz): 0,85 (m, 9H) ; 1,28 (m, 12H) ; 1,59 (m, 6H) ; 2,52 (m, 6H) ; 6,82 (t, 2,2 Hz, 1H) ; 7,07 (d, 8,8 Hz, 2H) ; 7,16 (d, 16,7 Hz, 1H) ; 7,21 (d, 2,2 Hz, 2H) ; 7,28 (d, 16,7 Hz, 1H) ; 7,57 (d, 8,8 Hz, 2H).

### Exemple 6 : Synthèse de 3,5-diacétate-4'-caproate de resvératrol (6)

Le composé 6 est synthétisé comme décrit à l'exemple 1, en remplacent le chlorure d'acide lipoïque par le chlorure d'hexanoyle (caproyle). On charge le diacétate de resvératrol (4 g, 12,8 mmol) qu'on solubilise dans 60 mL de THF anhydre. On ajoute goutte à goutte la triéthylamine (1,5 eq), puis le chlorure d'hexanoyle (2,72 mL ; 1,5 eq) dans un bain de glace (0 °C). Après 17 h d'agitation à température ambiante, le milieu est transféré dans une ampoule à décanter, lavé avec 10 mL d'une solution saturée de bicarbonate, puis extrait avec trois fois 20 mL d'acétate d'éthyle. La phase organique est lavée avec trois fois 20 mL de l'eau, puis séchée sur MgSO₄ anhydre. Le produit brut est obtenu après concentration sous pression réduite avec un rendement de 90 % et une pureté massique de 80 %.
¹H (CDCl₃, HMDS, 300 MHz): 0,87 (m, 3H) , 1,2-1,3 (m, 4H) ; 1,69 (m, 2H) ; 2,23 (s, 6H) ; 2,48 (t, 7,7 Hz, 2H) ; 6,75 (t, 1,9 Hz, 1 H) ; 6,89 (d, 15,9 Hz, 1 H) ; 6,99 (d, 15,9 Hz, 1 H) ; 7,01 (d, 8,5 Hz, 2H) ; 7,04 (d, 1,9 Hz, 2H) ; 7,41 (d, 8,5 Hz, 2H).

### Exemple comparatif 7 : Synthèse de 3,5,4'-trilipoate de resvératrol (Res(Lipoate)₃)

Le trilipoate de resvératrol est obtenu *via* le chlorure d'acide lipoïque préparé *in situ* sous argon à température ambiante dans le dichlorométhane. La réaction d'acylation conduit à un mélange des produits de couplage (monolipoates phénolique et résorcinique, dilipoates et trilipoates). Les trilipoates ont été obtenus après une chromatographie sur silice. Le produit n'a pas été caractérisé pour des raisons d'insolubilité. Pour les mêmes raisons il ne peut pas être bio-hydrolysé (voir tableau 1).

### Exemple 8 : Préparation d'extrait enzymatique

Les enzymes utilisées dans les essais de bio-hydrolyse *in vitro* ont été obtenues par la méthode de « tape stripping » comme décrit dans la littérature (Anal. Biochem., 290*,*

(2001), 179-185 ; Skin Pharmacol. Appl. Skin Physiol., 12, (1999), 182-192). Les échantillons de *Stratum corneum* sont prélevés avec le sparadrap chirurgical (type Bienderm 3M Health Care, St. Paul, Minnesota, USA). Les tampons utilisés dans les essais de bio-hydrolyse sont: 50 mM Tris (tris[hydroxyméthyl]aminométhane) pH 7,3 et pH 8 ; 50 mM Na-acétate pH 5,5 ; 50 mM MES (acide 2-[N-morpholino]-éthanesulfonique) pH 6 et 50 mM phosphate pH 6,5.

### Exemple 9 : Bio-hydrolyse

### a) Bio-hydrolyse avec les enzymes cutanées obtenues par tape-stripping

Les précurseurs sont solubilisés dans l'acétonitrile à concentration 1 g/L. Pour les essais de bio-hydrolyse on ajoute 50 µL de substrat et 50 µL d'acétonitrile dans 900 µL d'extrait enzymatique dans le tampon à pH choisi. Les mélanges réactionnels sont incubés à 35°C sans agitation et à l'abri de la lumière. L'évolution de la bio-hydrolyse est déterminée par HPLC (chromatographie liquide haute performance) à polarité de phase inversée. Les témoins sont réalisés dans les mêmes solutions sans extraits enzymatiques de la peau, pour déterminer la stabilité chimique des produits.

### b) Bio-hydrolyse avec la cholestérol estérase animale

L'enzyme utilisée est la cholestérol estérase de pancréas bovin, de la classe E.C. 3.1.1.13 (SIGMA C-3766). Les précurseurs sont préparés comme décrit à l'exemple 9a.

Les résultats sont présentés dans le Tableau 1 ci-dessous.

**Tableau 1**

| **Substrat (Précurseur)** | **Enzyme** | **Temps de bio-hydrolyse (h)** | **Taux de bio-hydrolyse (%)** | **Actif(s) libéré(s) par bio- hydrolyse** | | |
|---|---|---|---|---|---|---|
| | | | | **Polyphénol (PP)** | **A** | **B** |
| Res(caproate)₃ | *Stratum corneum* | 168 | 98 | Resvératrol | Caproate | |
| Res(Ac)₂=caproate | *Stratum corneum* | 72 | 95 | Resvératrol | Acétate + Caproate | |
| Res(lipoate)₃ * | * | | | | | |
| Res(AC)₂-lipoate | *Stratum corner* | 48 | 87 | Resvératrol + acide lipoïque | Acétate | Lipoate |
| Res(Ac)₂-succinate-Vit E** | *Stratum corneum* | 96 | 26 | Res-succinate-Vit E** | Acétate | |
| Res(Ac)₂-succinate-Vit E | Cholestérol estérase animale | 2 | 80 | Resvératrol | Acétate + Succinate | Vitamine E |
| | | 24 | 99 | | | |
| Res(Ac)₂-férulate(Ac) | *stratum corneum* | 6,5 | 71 | Resvératrol | Acétate | Acide férulique |
| Lut(Ac)₃-succinate-Vit E | *Stratum corneum* | 5 | 70 | Lutéoline | Acétate + Succinate | Vitamine E |

| | | | | | | |
|---|---|---|---|---|---|---|
| * Réaction non conduite car précurseur insoluble dans le milieu ** Les lipases du *Stratum comeum* hydrolysent les groupement acétyles du précurseur et conduisent à un intermédiaire (Res-succinate-Vit E) qui lui-même possède une activité anti-oxydante grâce aux groupement OH de la partie resvératrol di-acétylés | | | | | | |

Des estérases sont présentes naturellement dans le *Stratum granulosum.* Les exemples avec le Res(Ac)₂-succinate-Vit E montrent le potentiel de libération des actifs au niveau de la peau, avec l'action des enzymes du *Stratum corneum* sur les groupements acétates, permettant de libérer rapidement le pouvoir antioxydant des groupements alcools, puis l'action des enzymes du *Stratum granulosum* sur la séparation des actifs.

### Exemple 10 : Formulation Huile dans Eau

On réalise une formulation de Res(Ac)₂-Lipoate (Composé de l'exemple 1 selon l'invention) à 1% de type huile-dans-eau possédant la composition suivante (les pourcentages sont exprimés en poids) :

| | |
|---|---|
| Eau | 78,90 |
| EDTA tétrasodique | 0,10 |
| 1,3-butylène glycol | 5,00 |
| Emulium Delta^{® 1)} | 4,00 |
| Palmitate d'éthylhexyle | 5,00 |
| Alcool cétylique | 0,20 |
| Diméthicone | 3,00 |
| Phénonip^{® 2)} | 1,00 |
| Acétate de 2-DL-tocophérol | 0,30 |
| Res(Ac)₂-Lipoate selon l'invention | 1,00 |
| Acide citrique à 50% | 0,08 |
| Eau . | 0,92 |

| | |
|---|---|
| 1) Emulium Delta^{®} : alcool cétylique + stéarate de glycéryle + stéarate de PEG-75 + ceteth-20 + stéareth-20. 2) Phénonip^{®} : phénoxyéthanol + méthylparaben + éthylparaben + butylparaben + propylparaben + isobutylparaben. | |

La formulation ainsi obtenue présente une bonne stabilité après 56 jours à l'étuve à 45°C.

### Exemple 11 : Exemples de formulations

Les formulations suivantes sont fournies à titre purement illustratif. Les % sont exprimés en poids, par rapport au poids total de la composition.

### Formulation 11.1 : Formulation anhydre

| **Dénomination INCI** | **Quantité** |
|---|---|
| SODIUM SURFACTIN | 0,50 |
| GLYCERIN | 24,90 |
| 1,3 BUTYLENE GLYCOL | 7,50 |
| CYCLOMETHICONE 5 | 14,00 |
| DIMETHICONE 5 | 10,00 |
| ISONANOATE ISONONYLE | 10,00 |
| LIMNANTHES ALBA | 10,00 |
| ISODODECANE | 13,00 |
| CETIOL CC | 10,00 |
| Bioprécurseur selon l'exemple 1 | 0,10 |
| **Total** | **100,00** |

### Formulation 11.2 : Formulation Eau dans Huile (E/H)

| **Dénomination INCI** | **Quantité** |
|---|---|
| ETHYLHEXYL PALMITATE | 14,00 |
| PROPYLPARABEN | 0,30 |
| PEG-30 DIPOLYHYDROXYSTEARATE | 3,00 |
| SILICA DIMETHYL SILYLATE | 2,00 |
| HYDROGENATED POLYISOBUTENE | 31,49 |
| ETHYLENE/PROPYLENE/STYRENE COPOLYMER | 2,62 |
| BUTYLENE/ETHYLENE/STYRENE COPOLYMER | 0,88 |
| BHT | 0,01 |
| AQUA | 42,10 |
| SODIUM CHLORIDE | 0,80 |
| TETRASODIUM EDTA | 0,05 |
| GLYCERIN | 2,00 |
| METHYLPARABEN | 0,30 |
| XANTHAN GUM | 0,35 |
| Bioprécurseur selon l'exemple 1 | 0,10 |
| **Total** | **100,00** |

### Formulation 11,3 : Formulation E/Si

| **Dénomination INCI** | **Quantité** |
|---|---|
| CYCLOPENTASILOXANE | 24,25 |
| PEG/PPG-20/15 DIMETHICONE | 1,00 |
| DIMETHICONE | 5,00 |
| C₃₀₋₄₅ ALKYL CETEARYL DIMETHICONE CROSSPOLYMER | 2,25 |
| TOCOPHERYLACETATE | 0,30 |
| PHENOXYETHANOL | 0,72 |
| METHYLPARABEN | 0,16 |
| ETHYLPARABEN | 0,04 |
| BUTYLPARABEN | 0,04 |
| PROPYLPARABEN | 0,02 |
| ISOBUTYLPARABEN | 0,02 |
| AQUA (WATER) | 58,10 |
| SODIUM CHLORIDE | 1,00 |
| POLYSORBATE-20 | 1,00 |
| BUTYLENE GLYCOL | 3,00 |
| GLYCERIN | 3,00 |
| Bioprécurseur selon l'exemple 1 | 0,10 |
| **Total** | **100,00** |

### Formulation 11.4 : Formulation de type sérum

| **Dénomination INCI** | **Quantité** |
|---|---|
| AQUA (WATER) | 85,19 |
| TETRASODIUM EDTA | 0,05 |
| POLYETHYLENEGLYCOL | 5,00 |
| ACRYLATES/C10-30 ALKYL ACRYLATE CROSSPOLYMER | 0,35 |
| AMMONIUM ACRYLOYLDIMETHYLTAURAT/VP COPOLYMER | 0,30 |
| GLYCERIN | 4,43 |
| PEG-8 | 1,00 |
| SODIUM POLYACRYLATE | 0,04 |
| CAPRYLYL GLYCOL | 0,15 |
| PEG-11 METHYL ETHER DIMETHICONE | 3,00 |
| PROPYLENE GLYCOL | 0,01 |
| PROPYLPARABEN | 0,01 |
| METHYLPARABEN | 0,31 |
| SODIUM HYDROXIDE | 0,06 |
| Bioprécurseur selon l'exemple 1 | 0,10 |
| **Total** | **100,00** |

### Formulation 11.5 : Formulation de type sérum E/Si

| **Dénomination INCI** | **Quantité** |
|---|---|
| CYCLOPENTASILOXANE | 28,97 |
| PEG/PPG-18/188 METHICONE | 0,90 |
| CYCLOHEXASILOXANE | 4,38 |
| SORBITAN SESQUIOLEATE | 0,50 |
| PEG-45/DODECYL GLYCOL COPOLYMER | 1,00 |
| ISOCETYL STEARATE | 1,00 |
| HYDROGENATED CASTOR OIL | 0,25 |
| C30-45 ALKYL CETEARYL DIMETHICONE CROSSPOLYMER | 2,25 |
| BHT | 0,01 |
| ASCORBYL PALMITATE | 0,01 |
| GLYCERYL STEARATE | 0,01 |
| GLYCERIN | 1,50 |
| PROPYLENE GLYCOL | 1,50 |
| AQUA (WATER) | 48,06 |
| TETRASODIUM EDTA | 0,05 |
| SCLEROTIUM GUM | 0,20 |
| SODIUM CHLORIDE | 0,80 |
| HDI/TRIMETHYLOL HEXYLLACTONE CROSSPOLYMER | 2,45 |
| SILICA | 2,55 |
| ALCOHOL DENAT | 3,00 |
| METHYLPARABEN | 0,25 |
| CHLORPHENESIN | 0,26 |
| Bioprécurseur selon l'exemple 1 | 0,10 |
| **Total** | **100,00** |

### Formulation 11.6 : Formulation Gel Hydro-alcoolique

| **Dénomination INCI** | **Quantité** |
|---|---|
| AQUA (WATER) | 56,95 |
| TETRASODIUM EDTA | 0,05 |
| ACRYLATES/VINYL ISODECANOATE CROSSPOLYMER | 0,50 |
| GLYCERIN | 5,00 |
| PROPYLENE GLYCOL | 8,00 |
| ACRYLATES/C₁₀₋₃₀ ALKYL ACRYLATE CROSSPOLYMER | 0,20 |
| PENTYLENE GLYCOL | 6,00 |
| CYCLOPENTASILOXANE | 6,00 |
| DIMETHICONE | 1,74 |
| DIMETHICONOL | 0,26 |
| ALCOHOL DENAT | 15,00 |
| TETRAHYDROXYPROPYL ETHYLENEDIAMINE | 0,20 |
| Bioprécurseur selon l'exemple 1 | 0,10 |
| **Total** | **100,00** |

### Formulation 11.7 : Formulation Gel - Crème

| **Dénomination INCI** | **Quantité** |
|---|---|
| AQUA (WATER) | 75,36 |
| TETRASODIUM EDTA | 0,05 |
| GLYCERIN | 7,26 |
| AMMONIUM ACRYLOYLDIMETHYLTAURATE/VP COPOLYMER | 0,80 |
| ACRYLATES/C10-30 ALKYL ACRYLATE CROSSPOLYMER | 0,15 |
| METHYLPARABEN | 0,30 |
| CYCLOTETRASILOXANE | 0,02 |
| POLYSORBATE-20 | 0,05 |
| ISONONYL ISONANOATE | 3,00 |
| CYCLOPENTASILOXANE | 5,23 |
| CYCLOHEXASILOXANE | 2,80 |
| DIMETHICONE | 2,00 |
| PHENYL TRIMETHICONE | 2,00 |
| TOCOPHERYL ACETATE | 0,50 |
| POLYACRYLAMIDE | 0,12 |
| HYDROGENATED POLYISOBUTENE | 0,05 |
| LAURETH-7 | 0,02 |
| HYDROXYETHYL ACRYLATE/SODIUM ACRYLOYLDIMETHYL TAURATE COPOLYMER | 0,09 |
| SQUALANE | 0,06 |
| POLYSORBATE 60 | 0,01 |
| SORBITAN ISOSTEARATE | 0,01 |
| SODIUM HYDROXIDE | 0,02 |
| Bioprécurseur selon l'exemple 1 | 0,10 |
| **Total** | **100,00** |

### Formulation 11.8 : Formulation anhydre

| **Dénomination INCI** | **Quantité** |
|---|---|
| SODIUM SURFACTIN | 0,50 |
| GLYCERIN | 24,90 |
| 1,3 BUTYLENE GLYCOL | 7,50 |
| CYCLOMETHICONE 5 | 14,00 |
| DIMETHICONE 5 | 10,00 |
| ISONANOATE ISONONYLE | 10,00 |
| LIMNANTHES ALBA | 10,00 |
| ISODODECANE | 13,00 |
| CETIOL CC | 10,00 |
| Bioprécurseur selon l'exemple 3 | 0,10 |
| **Total** | **100,00** |

### Formulation 11.9: Formulation Eau dans Huile (E/H)

| **Dénomination INCI** | **Quantité** |
|---|---|
| ETHYLHEXYL PALMITATE | 14,00 |
| PROPYLPARABEN | 0,30 |
| PEG-30 DIPOLYHYDROXYSTEARATE | 3,00 |
| SILICA DIMETHYL SILYLATE | 2,00 |
| HYDROGENATED POLYISOBUTENE | 31,49 |
| ETHYLENE/PROPYLENE/STYRENE COPOLYMER | 2,62 |
| BUTYLENE/ETHYLENE/STYRENE COPOLYMER | 0,88 |
| BHT | 0,01 |
| AQUA | 42,10 |
| SODIUM CHLORIDE | 0,80 |
| TETRASODIUM EDTA | 0,05 |
| GLYCERIN | 2,00 |
| METHYLPARABEN | 0,30 |
| XANTHAN GUM | 0,35 |
| Bioprécurseur selon l'exemple 3 | 0,10 |
| **Total** | **100,00** |

### Formulation 11.10 : Formulation E/Si

| **Dénomination INCI** | **Quantité** |
|---|---|
| CYCLOPENTASILOXANE | 24,25 |
| PEG/PPG-20/15 DIMETHICONE | 1,00 |
| DIMETHICONE | 5,00 |
| C₃₀₋₄₅ ALKYL CETEARYL DIMETHICONE CROSSPOLYMER | 2,25 |
| TOCOPHERYLACETATE | 0,30 |
| PHENOXYETHANOL | 0,72 |
| METHYLPARABEN | 0,16 |
| METHYLPARABEN | 0,04 |
| BUTYLPARABEN | 0,04 |
| PROPYLPARABEN | 0,02 |
| ISOBUTYLPARABEN | 0,02 |
| AQUA (WATER) | 58,10 |
| SODIUM CHLORIDE | 1,00 |
| POLYSORBATE-20 | 1,00 |
| BUTYLENE GLYCOL | 3,00 |
| GLYCERIN | 3,00 |
| Bioprécurseur selon l'exemple 3 | 0,10 |
| **Total** | **100,00** |

### Formulation 11.11 : Formulation de type sérum

| **Dénomination INCI** | **Quantité** |
|---|---|
| AQUA (WATER) | 85,19 |
| TETRASODIUM EDTA | 0,05 |
| POLYETHYLENEGLYCOL | 5,00 |
| ACRYLATES/C10-30 ALKYL ACRYLATE CROSSPOLYMER | 0,35 |
| AMMONIUM ACRYLOYLDIMETHYLTAURATE/VP COPOLYMER | 0,30 |
| GLYCERIN | 4,43 |
| PEG-8 | 1,00 |
| SODIUM POLYACRYLATE | 0,04 |
| CAPRYLYL GLYCOL | 0,15 |
| PEG-11 METHYL ETHER DIMETHICONE | 3,00 |
| PROPYLENE GLYCOL | 0,01 |
| PROPYLPARABEN | 0,01 |
| METHYLPARABEN | 0,31 |
| SODIUM HYDROXIDE | 0,06 |
| Bioprécurseur selon l'exemple 3 | 0,10 |
| **Total** | **100,00** |

### Formulation 11.12 : Formulation de type sérum E/Si

| **Dénomination INCI** | **Quantité** |
|---|---|
| CYCLOPENTASILOXANE | 28,97 |
| PEG/PPG-18/18 DIMETHICONE | 0,90 |
| CYCLOHEXASILOXANE | 4,38 |
| SORBITAN SESQUIOLEATE | 0,50 |
| PEG-45/DODECYL GLYCOL COPOLYMER | 1,00 |
| ISOCETYL STEARATE | 1,00 |
| HYDROGENATED CASTOR OIL | 0,25 |
| C30-4.5 ALKYL CETEARYL DIMETHICONE CROSSPOLYMER | 2,25 |
| BHT | 0,01 |
| ASCORBYL PALMITATE | 0,01 |
| GLYCERYL STEARATE | 0,01 |
| GLYCERIN | 1,50 |
| DROPYLENE GLYCOL | 1,50 |
| AQUA (WATER) | 48,06 |
| TETRASODIUM EDTA | 0,05 |
| SCLEROTIUM GUM | 0,20 |
| SODIUM CHLORIDE | 0,80 |
| HDI/TRIMETHYLOL HEXYLLACTONE CROSSPOLYMER | 2,45 |
| SILICA | 2,55 |
| ALCOHOL DENAT | 3,00 |
| METHYLPARABEN | 0,25 |
| CHLORPHENESIN | 0,26 |
| Bioprécurseur selon l'exemple 3 | 0,10 |
| **Total** | **100,00** |

### Formulation 11.13 : Formulation Gel Hydro-alcoolique

| **Dénomination INCI** | **Quantité** |
|---|---|
| AQUA (WATER) | 56,95 |
| TETRASODIUM EDTA | 0,05 |
| ACRYLATES/VINYL ISODECANOATE CROSSPOLYMER | 0,50 |
| GLYCERIN | 5,00 |
| PROPYLENE GLYCOL | 8,00 |
| ACRYLATES/C₁₀₋₃₀ ALKYL ACRYLATE CROSSPOLYMER | 0,20 |
| PENTYLENE GLYCOL | 6,00 |
| CYCLOPENTASILOXANE | 6,00 |
| DIMETHICONE | 1,74 |
| DIMETHICONOL | 0,26 |
| ALCOHOL DENAT | 15,00 |
| TETRAHYDROXYPROPYL ETHYLENEDIAMINE | 0,20 |
| Bioprécurseur selon l'exemple 3 | 0,10 |
| **Total** | **100,00** |

### Formulation 11.14 : Formulation Gel - Crème

| **Dénomination INCI** | **Quantité** |
|---|---|
| AQUA (WATER) | 75,36 |
| TETRASODIUM EDTA | 0,05 |
| GLYCERIN | 7,26 |
| AMMONIUM ACRYLOYLDIMETHYLTAURATE/VP COPOLYMER | 0,80 |
| ACRYLATES/C10-30 ALKYL ACRYLATE CROSSPOLYMER | 0,15 |
| METHYLPARABEN | 0,30 |
| CYCLOTETRASILOXANE | 0,02 |
| POLYSORBATE-20 | 0,05 |
| ISONONYL ISONANOATE | 3,00 |
| CYCLOPENTASILOXANE | 5,23 |
| CYCLOHEXASILOXANE | 2,80 |
| DIMETHICONE | 2,00 |
| PHENYL TRIMETHICONE | 2,00 |
| TOCOPHERYLACETATE | 0,50 |
| POLYACRYLAMIDE | 0,12 |
| HYDROGENATED POLYISOBUTENE | 0,05 |
| LAURETH-7 | 0,02 |
| HYDROXYETHYL ACRYLATE/SODIUM ACRYLOYLDIMETHYL TAURATE COPOLYMER | 0,09 |
| SQUALANE | 0,06 |
| POLYSORBATE 60 | 0,01 |
| SORBITAN ISOSTEARATE | 0,01 |
| SODIUM HYDROXIDE | 0,02 |
| Bioprécurseur selon l'exemple 3 | 0,10 |
| **Total** | **100,00** |

Il doit être bien compris que l'invention définie par les revendications annexées n'est pas limitée aux modes de réalisation particuliers indiqués dans la description ci-dessus.

## Revendications

1. Utilisation d'un bioprécurseur répondant à la structure :
[A]ₙ -PP- [B]ₘ
dans laquelle :
- PP représente un reste d'un polyphénol où chaque fonction hydroxyle est protégée par un groupement A ou un groupement B ;
- A est une chaîne alkyle substituée ou non, saturée ou insaturée, comprenant de 1 à 20 atomes de carbone, préférentiellement de 1 à 4, qui est liée au polyphénol par :
- une fonction ester carboxylique sur une fonction hydroxyle dudit polyphénol ; ou
- l'intermédiaire d'un espaceur A', dans lequel A est lié à A' par une fonction ester carboxylique, et A' est lié au polyphénol par une fonction ester carboxylique sur une fonction hydroxyle dudit polyphénol ;
- n représente un entier supérieur ou égal à 1, notamment 1, 2, 3, 4 ou 5 ;
- B est un précurseur d'une molécule biologiquement active, qui est lié au polyphénol par :
- une fonction ester carboxylique sur une fonction hydroxyle dudit polyphénol ; ou
- l'intermédiaire d'un espaceur B', dans lequel B est lié à B' par une fonction ester carboxylique, et B' est lié au polyphénol par une fonction ester carboxylique sur une fonction hydroxyle dudit polyphénol ;
ladite molécule biologiquement active étant choisie parmi : une molécule cosmétiquement active, une molécule pharmaceutiquement active, une molécule dermatologiquement active
ladite molécule cosmétiquement active, pharmaceutiquement active ou dermatologiquement active étant choisie parmi : polyphénol, acide lipoïque, vitamines A, B, D, E, F, acides rétinoïques,
- m représente un entier supérieur ou égal à 1, notamment 1 ou 2,
le groupement A étant différent du groupement B
dans une formulation topique, pour la préparation d'une composition thérapeutique, dermatologique ou cosmétique.

2. Utilisation selon la revendication 1, **caractérisé en ce que** le polyphénol PP est du type comportant au moins 2 noyaux phénols.

3. Utilisation selon la revendication 1, **caractérisé en ce que** le polyphénol PP répond à la formule suivante : 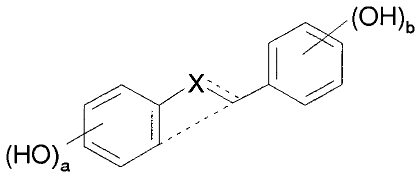 dans laquelle :
• a est 1,2,3,4 ou 5
• b est 1,2,3,4 ou 5
• X est N, S, O, CH, CH₂, CO, NH,
• ----représente une double ou une simple liaison
• ---- représente une chaîne, éventuellement présente, formant un cycle à 5 ou 6 chaînons, incluant X et comportant 1 ou plusieurs doubles liaisons, éventuellement un ou plusieurs substituants OH et/ou un ou plusieurs hétéroatomes choisis parmi N, S, O, situés dans le cycle et/ou en substituant.

4. Utilisation selon la revendication 1, **caractérisé en ce que** le polyphénol PP répond à la formule suivante : 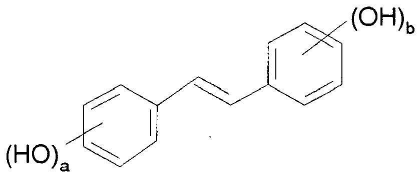 dans laquelle a est 1, 2, 3, 4 ou 5 et b est 1, 2, 3, 4 ou 5,.

5. Utilisation selon la revendication 4, **caractérisé en ce que** a est 2 et b est 1.

6. Utilisation selon la revendication 1, **caractérisé en ce que** le polyphénol PP répond à la formule suivante : 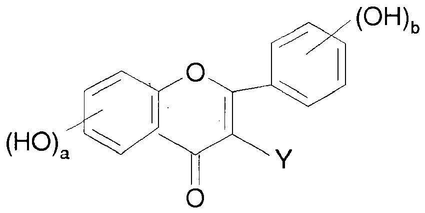 dans laquelle Y est H ou OH, a est 1, 2, 3 ou 4 et b est 1, 2, 3, 4 ou 5.

7. Utilisation selon la revendication 4, **caractérisé en ce que** a est 2 et b est 2.

8. Utilisation selon la revendication 1, **caractérisé en ce que** le polyphénol PP est choisi parmi : resvératrol, lutéoline, quercétine, hydroquinone, pyrocatéchol, acide gallique, hydroxytyrosol, tétrahydrocurcumin, silylmarin, acide ellagique.

9. Utilisation l'une quelconque des revendications précédentes, **caractérisé en ce que** le groupement A du bioprécurseur est issu d'un acide carboxylique linéaire, ramifié ou cyclique, choisi parmi l'acide éthanoïque, l'acide propanoïque, l'acide butanoïque linéaire ou ramifié, l'acide caproïque et l'acide laurique.

10. Utilisation selon l'une quelconque des revendications précédentes, **caractérisé en ce que** les groupements A' et B' du bioprécurseur sont, indépendamment l'un de l'autre, une chaîne hydrocarbonée de préférence aliphatique comprenant deux fonctions acides ou une fonction acide et une fonction hydroxyle comprenant de 2 à 13 atomes de carbone, de préférence de 2 à 5.

11. Utilisation selon la revendication 10, **caractérisé en ce que** A' et B' sont indépendamment l'un de l'autre un reste d'acide succinique, d'acide adipique, d'acide brassylique, d'acide lactique, d'acide salicylique, d'acide 4-hydroxybenzoïque, d'acide férulique, d'acide tartrique, d'acide 2-hydroxybutanoïque, d'acide 3-hydroxybutanoïque, d'acide 4-hydroxybutanoïque.

12. Utilisation selon la revendication 1, dans laquelle le bioprécurseur est choisi parmi :
- (E)-4-(3,5-diacétoxystyryl)phényl-5-(1,2-dithiolan-3-yl)pentanoate ;
- (E)-4-(3,5-diacétoxystyryl)phényl-2,5,7,8-tétraméthyl-2-(4,8,12-triméthyltridécyl)-3,4-dihydro-2H-chromen-6-ylsuccinate ;
- 4-((E)-3,5-diacétoxystyryl)phényl-(2Z,4E,6Z,8E)-3,7-diméthyl-9-(2,6,6-triméthylcyclohex-1-enyl)nona-2,4,6,8-tétraényl succinate ;
- (2E,4E,6E,8E,10E,12E)-4-((E)-3,5-diacétoxystyryl)phényl docosa-2,4,6,8,10,12-hexaénoate ;
- (E)-4-(3,5-diacétoxystyryl)phényl 4-hydroxy-3-méthoxybenzoate ;
- (E)-4-(3,5-diacétoxystyryl)phényl 3,4-diacétoxybenzoate ;
- (E)-4-((E)-3,5-diacétoxystyryl)phényl 3-(4-hydroxy-3-méthoxyphényl)acrylate ;
- (E)-4-((E)-3,5-diacétoxystyryl)phényl 3-(4-acétoxy-3-méthoxyphényl)acrylate ;
- (E)-4-((E)-3,5-diacétoxystyryl)phényl 3-(4-méthoxyphényl)acrylate ;
- (E)-4-(3,5-diacétoxystyryl)phényl 2-acétoxybenzoate ;
- 7-acétoxy-2-(3,4-diacétoxyphényl)-4-oxo-4H-chromen-5-yl 5-(1,2-dithiolan-3-yl)pentanoate ;
- 7-acétoxy-2-(3,4-diacétoxyphényl)-4-oxo-4H-chromen-5-yl 2,5,7,8-tetraméthyl-2-(4,8,12-triméthyltridécyl)-3,4-dihydro-2H-chromen-6-yl succinate ;
- 2-méthoxy-4-(7-(3-méthoxy-4-(prop-1-en-2-yloxy)phényl)-3,5-dioxoheptyl)phényl 5-(1,2-dithiolan-3-yl)pentanoate ; et
- 2-méthoxy-4-(7-(3-méthoxy-4-(prop-1-en-2-yloxy)phényl)-3,5-dioxoheptyl)phényl 2,5,7,8-tétraméthyl-2-(4,8,12-triméthyltridécyl)-3,4-dihydro-2H-chromen-6-yl succinate.

13. Utilisation selon la revendication 12, dans laquelle le bioprécurseur est choisi parmi le pentanoate de (***E***)*-*4-(3,5-diacétoxystyryl)phényl-5-(1,2-dithiolan-3-yle) et le succinate de (*E*)-4-(3,5-diacétoxystyryl)phényl-2,5,7,8-tétraméthyl-2-(4,8,12-triméthyltridécyl)-3,4-dihydro-2*H-*chromen-6-yle.

14. Bioprécurseur répondant à la structure :
[A]ₙ - PP - [B]ₘ
dans laquelle :
- PP représente un reste d'un polyphénol où chaque fonction hydroxyle est protégée par un groupement A ou un groupement B ;
- A est une chaîne alkyle substituée ou non, saturée ou insaturée, comprenant de 1 à 20 atomes de carbone, préférentiellement de 1 à 4, qui est liée au polyphénol par :
- une fonction ester carboxylique sur une fonction hydroxyle dudit polyphénol ; ou
- l'intermédiaire d'un espaceur A', dans lequel A est lié à A' par une fonction ester carboxylique, et A' est lié au polyphénol par une fonction ester carboxylique sur une fonction hydroxyle dudit polyphénol ;
- n représente un entier supérieur ou égal à 1, notamment 1, 2, 3, 4 ou 5 ;
- B est un précurseur d'une molécule biologiquement active, qui est lié au polyphénol par :
- une fonction ester carboxylique sur une fonction hydroxyle dudit polyphénol ; ou
- l'intermédiaire d'un espaceur B', dans lequel B est lié à B' par une fonction ester carboxylique, et B' est lié au polyphénol par une fonction ester carboxylique sur une fonction hydroxyle dudit polyphénol ;
ladite molécule biologiquement active étant choisie parmi : une molécule cosmétiquement active, une molécule pharmaceutiquement active ou une molécule dermatologiquement active choisie parmi : polyphénol, acide lipoïque, vitamines A, B, D, E, F, acides rétinoïques,
- m représente un entier supérieur ou égal à 1, notamment 1 ou 2,
le groupement A étant différent du groupement B.

15. Bioprécurseur selon la revendication 14, **caractérisé en ce que** le polyphénol PP est du type comportant au moins 2 noyaux phénols.

16. Bioprécurseur selon la revendication 14, **caractérisé en ce que** le polyphénol PP répond à la formule suivante : 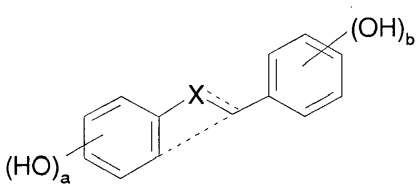 dans laquelle :
• a est 1,2,3,4 ou 5
• b est 1,2,3,4 ou 5
• X est N, S, O, CH, CH₂, CO, NH,
• ----représente une double ou une simple liaison
• ---- représente une chaîne, éventuellement présente, formant un cycle à 5 ou 6 chaînons, incluant X et comportant 1 ou plusieurs doubles liaisons, éventuellement un ou plusieurs substituants OH et/ou un ou plusieurs hétéroatomes choisis parmi N, S, O, situés dans le cycle et/ou en substituant.

17. Bioprécurseur selon la revendication 14, **caractérisé en ce que** le polyphénol PP répond à la formule suivante : 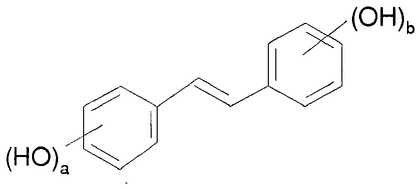 dans laquelle a est 1, 2, 3, 4 ou 5 et b est 1, 2, 3, 4 ou 5,.

18. Bioprécurseur selon la revendication 17, **caractérisé en ce que** a est 2 et b est 1.

19. Bioprécurseur selon la revendication 14, **caractérisé en ce que** le polyphénol PP répond à la formule suivante : 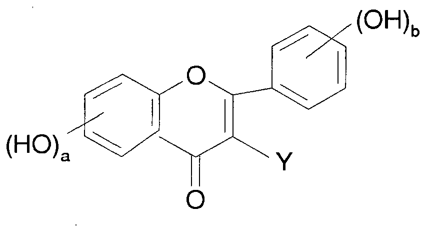 dans laquelle Y est H ou OH, a est 1, 2, 3 ou 4 et b est 1, 2, 3, 4 ou 5.

20. Bioprécurseur selon la revendication 19, **caractérisé en ce que** a est 2 et b est 2.

21. Bioprécurseur selon la revendication 14, **caractérisé en ce que** le polyphénol PP est choisi parmi : resvératrol, lutéoline, quercétine, hydroquinone, pyrocatéchol, acide gallique, hydroxytyrosol, tétrahydrocurcumin, silylmarin, acide ellagique.

22. Bioprécurseur selon l'une quelconque des revendications 14 à 21, **caractérisé en ce que** A est issu d'un acide carboxylique linéaire, ramifié ou cyclique, choisi parmi l'acide éthanoïque, l'acide propanoïque, l'acide butanoïque linéaire ou ramifié, l'acide caproïque et l'acide laurique.

23. Bioprécurseur selon l'une quelconque des revendications 14 à 21, **caractérisé en ce que** A' et B' sont indépendamment l'un de l'autre une chaîne hydrocarbonée de préférence aliphatique comprenant deux fonctions acides ou une fonction acide et une fonction hydroxyle comprenant de 2 à 13 atomes de carbone, de préférence de 2 à 5.

24. Bioprécurseur selon la revendication 23, **caractérisé en ce que** A' et B' sont indépendamment l'un de l'autre un reste d'acide succinique, d'acide adipique, d'acide brassylique, d'acide lactique, d'acide salicylique, d'acide 4-hydroxybenzoïque, d'acide férulique, d'acide tartrique, d'acide 2-hydroxybutanoïque, d'acide 3-hydroxybutanoïque, d'acide 4-hydroxybutanoïque.

25. Bioprécurseur selon la revendication 14, choisi parmi :
- pentanoate de (E)-4-(3,5-diacétoxystyryl)phényl-5-(1,2-dithiolan-3-yle) ;
- succinate de (E)-4-(3,5-diacétoxystyryl)phényl-2,5,7,8-tétraméthyl-2-(4,8,12-triméthyltridécyl)-3,4-dihydro-2*H*-chromén-6-yle ;
- succinate de 4-((E)-3,5-diacétoxystyryl)phényl (2Z,4E,6Z,8E)-3,7-diméthyl-9-(2,6,6-triméthylcyclohex-1-enyl)nona-2,4,6,8-tétraényle ;
- (2E,4E,6E,8E,10E,12E)-4-((E)-3,5-diacétoxystyryl)phényl docosa-2,4,6,8,10,12-hexaénoate ;
- (E)-4-(3,5-diacétoxystyryl)phényl 4-hydroxy-3-méthoxybenzoate ;
- (E)-4-(3,5-diacétoxystyryl)phényl 3,4-diacétoxybenzoate ;
- (E)-4-((E)-3,5-diacétoxystyryl)phényl 3-(4-hydroxy-3-méthoxyphényl)acrylate ;
- (E)-4-((E)-3,5-diacétoxystyryl)phényl 3-(4-acétoxy-3-méthoxyphényl)acrylate ;
- (E)-4-((E)-3,5-diacétoxystyryl)phényl 3-(4-méthoxyphényl)acrylate ;
- (E)-4-(3,5-diacétoxystyryl)phényl 2-acétoxybenzoate ;
- 7-acétoxy-2-(3,4-diacétoxyphényl)-4-oxo-4H-chromen-5-yl 5-(1,2-dithiolan-3-yl)pentanoate ;
- 7-acétoxy-2-(3,4-diacétoxyphényl)-4-oxo-4H-chromen-5-yl 2,5,7,8-tetraméthyl-2-(4,8,12-triméthyltridécyl)-3,4-dihydro-2H-chromen-6-yl succinate ;
- 2-méthoxy-4-(7-(3-méthoxy-4-(prop-1-en-2-yloxy)phényl)-3,5-dioxoheptyl)phényl 5-(1,2-dithiolan-3-yl)pentanoate ; et
- 2-méthoxy-4-(7-(3-méthoxy-4-(prop-1-en-2-yloxy)phényl)-3,5-dioxoheptyl)phényl 2,5,7,8-tétraméthyl-2-(4,8,12-triméthyltridécyl)-3,4-dihydro-2H-chromen-6-yl succinate.

26. Bioprécurseur selon la revendication 25, choisi parmi le pentanoate de (*E*)-4-(3,5-diacétoxystyryl)phényl-5-(1,2-dithiolan-3-yle) et le succinate de (*E*)-4-(3,5-diacétoxystyryl)phényl-2,5,7,8-tétraméthyl-2-(4,8,12-triméthyltridécyl)-3,4-dihydro-2*H-*chromen-6-yle.

27. Composition comprenant au moins un bioprécurseur selon l'une quelconque des revendications 14 à 26, comportant un précurseur B d'une molécule dermatologiquement ou cosmétiquement active, dans une formulation topique acceptable pour une application dermatologique ou cosmétique.

28. Composition comprenant au moins un bioprécurseur selon l'une quelconque des revendications 14 à 26, comportant un précurseur B d'une molécule thérapeutique, dans une formulation topique acceptable pour une application thérapeutique.

29. Procédé de traitement cosmétique, comprenant l'application sur la peau d'une composition cosmétique selon la revendication 27.

30. Procédé de fabrication d'un bioprécurseur selon l'une quelconque des revendications 14 à 26, comprenant au moins les étapes suivantes :
a) protection du polyphénol par per-estérification, par un composé A-Z, Z étant une fonction susceptible de réagir avec une fonction OH du polyphénol pour générer la liaison ester entre le polyphénol et A,
b) déprotection sélective de manière à obtenir une ou plusieurs fonctions OH libres du polyphénol, et
c) couplage de l'intermédiaire obtenu après l'étape b) avec la molécule biologiquement active B préalablement activée.

## Patentansprüche

1. Verwendung eines Bio-Precursors, der der Struktur entspricht:
[A]ₙ-PP-[B]ₘ
in der:
- PP einen Rest eines Polyphenols darstellt, bei dem jede Hydroxylfunktion durch eine Gruppe A oder eine Gruppe B geschützt ist;
- A eine substituierte oder nichtsubstituierte, gesättigte oder nichtgesättigte Alkylkette ist, die 1 bis 20, vorzugsweise 1 bis 4, Kohlenstoffatome umfasst, die an das Polyphenol gebunden ist durch:
- eine Ester-Carboxylfunktion an einer Hydroxylfunktion des Polyphenols; oder
- über einen Spacer A', bei dem A durch eine Ester-Carboxylfunktion an A' gebunden ist und A' an das Phenol durch eine Ester-Carboxylfunktion an eine Hydroxylfunktion des Phenols gebunden ist;
- n eine ganze Zahl größer oder gleich 1, insbesondere 1, 2, 3, 4 oder 5 darstellt;
- B ein Precursor eines biologisch aktiven Moleküls ist, das an das Polyphenol durch:
- eine Ester-Carboxylfunktion an eine Hydroxylfunktion des Phenols gebunden ist; oder
- über einen Spacer B', bei dem B durch eine Ester-Carboxylfunktion an B' gebunden ist, und B' an das Polyphenol durch eine Ester-Carboxylfunktion an eine Hydroxylfunktion des Polyphenols gebunden ist;
wobei das biologisch aktive Molekül ausgewählt ist aus: einem kosmetisch aktiven Molekül, einem pharmazeutisch aktiven Molekül, einem dermatologisch aktiven Molekül,
wobei das kosmetisch aktive Molekül, das pharmazeutisch aktive Molekül oder das dermatologisch aktive Molekül ausgewählt sind aus: Polyphenol, Lipoidsäure, Vitaminen A, B, D, E, F, Retinsäuren,
- m eine ganze Zahl größer oder gleich 1, insbesondere 1 oder 2, darstellt,
die Gruppe A unterschiedlich zur Gruppe B ist in einer topischen Formulierung für die Herstellung einer therapeutischen, dermatologischen oder kosmetischen Zusammensetzung.

2. Verwendung nach Anspruch 1, **dadurch gekennzeichnet, dass** das Polyphenol PP von einer Art ist, die mindestens 2 Phenolkerne aufweist.

3. Verwendung nach Anspruch 1, **dadurch gekennzeichnet, dass** das Polyphenol PP folgender Formel entspricht: 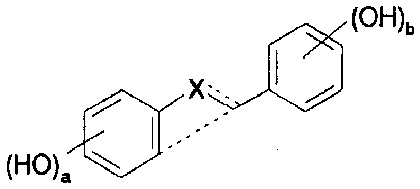 in der:
• a 1, 2, 3, 4 oder 5 ist
• b 1,2,3,4 oder 5 ist
• X N, S, O, CH, CH₂, CO, NH ist,
• ---- eine doppelte oder eine einfache Bindung darstellt
----
• eine gegebenenfalls vorhandene Kette darstellt, die einen Zyklus mit fünf oder sechs Kettengliedern bildet, die X einschließen und ein oder mehrere Doppelbindungen, gegebenenfalls ein oder mehrere Substituenten OH und/oder ein oder mehrere Heteroatome aufweisen, die ausgewählt sind aus N, S, O, die in dem Zyklus angeordnet sind und/oder als Substituent.

4. Verwendung nach Anspruch 1, **dadurch gekennzeichnet, dass** das Polyphenol PP folgender Formel entspricht: 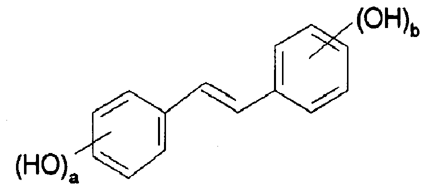 in der a 1, 2, 3, 4 oder 5 und b 1, 2, 3, 4 oder 5 ist.

5. Verwendung nach Anspruch 4, **dadurch gekennzeichnet, dass** a gleich 2 und b gleich 1 ist.

6. Verwendung nach Anspruch 1, **dadurch gekennzeichnet, dass** das Polyphenol PP folgender Formel entspricht: 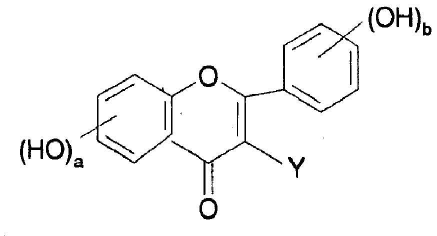 in der Y H oder OH ist, a 1, 2, 3 oder 4 und b 1, 2, 3, 4 oder 5 ist.

7. Verwendung nach Anspruch 4, **dadurch gekennzeichnet, dass** a 2 und b 2 ist.

8. Verwendung nach Anspruch 1, **dadurch gekennzeichnet, dass** das Polyphenol PP ausgewählt ist aus: Resveratrol, Luteolin, Quercetin, Hydroquinon, Pyrocatechol, Gallussäure, Hydroxytyrosol, Tetrahydrocurcumin, Silylmarin, Ellagsäure.

9. Verwendung nach einem beliebigen der vorhergehenden Ansprüche, **dadurch gekennzeichnet, dass** die Gruppe A des Bio-Precursors von einer linearen Carboxylsäure, verzweigt oder zyklisch, stammt, die ausgewählt ist aus Essigsäure, Propionsäure, Buttersäure, linear oder verzweigt, Capronsäure und Laurinsäure.

10. Verwendung nach beliebigen der vorhergehenden Ansprüche, **dadurch gekennzeichnet, dass** die Gruppen A' und B' des Bio-Precursors unabhängig voneinander eine Kohlenwasserstoffkette, vorzugsweise eine aliphatische Kohlenwasserstoffkette sind, die zwei Säurefunktionen oder eine Säurefunktion und eine Hydroxylfunktion aufweist, die 2 bis 13, vorzugsweise 2 bis 5 Kohlenstoffatome umfasst.

11. Verwendung nach Anspruch 10, **dadurch gekennzeichnet, dass** A' und B' unabhängig voneinander ein Bernsteinsäure-, Adipinsäure-, Brassylsäure-, Milchsäure-, Silicylsäure-, 4-Hydroxybenzoesäure-, Ferulasäure-, Apfelsäure-, 2-Hydroxybutansäure-, 3-Hydroxybutansäure-, 4-Hydroxybutansäurerest sind.

12. Verwendung nach Anspruch 1, bei der der Bio-Precursor ausgewählt ist aus:
- (E)-4-(3,5-Diacetoxystyryl)phenyl-5-(1,2-dithiolan-3-yl) pentanoat;
- (E)-4-(3,5-Diacetoxystyryl)phenyl-2,5,7,8-tetramethyl-2-(4,8,12-trimethyltridecyl)-3,4-dihydro-2H-chromen-6-yl-succinat;
- 4-((E)-3,5-Diacetoxystyryl)phenyl-(2Z,4E,6Z,8E)-3,7-dimethyl-9-(2,6,6-trimethylcyclohex-1-enyl)nona-2,4, 6,8-tetraenylsuccinat;
- (2E,4E,6E,8E, 10E,12E)-4-((E)-3,5-Diacetoxystyryl)phenyl docosa-2,4,6,8,10,12-hexaenoat;
- (E)-4-(3,5-Diacetoxystyryl)phenyl 4-hydroxy-3-methoxybenzoat;
- (E)-4-(3,5-Diacetoxystyryl)phenyl 3,4-diacetoxybenzoat;
- (E)-4-((E)-3,5-Diacetoxystyryl)phenyl 3-(4-hydroxy-3-Methoxyphenyl)acrylat;
- (E)-4-((E)- 3,5-Diacetoxystyryl) phenyl 3-(4-acetoxy-3-methoxyphenyl)acrylat;
- (E)-4-((E)-3,5-Diacetoxystyryl) phenyl 3-(4-methoxyphenyl)acrylat;
- (E)-4-(3,5-Diacetoxystyryl)phenyl 2-acetoxybenzoat;
- 7-Acetoxy-2-(3,4-diacetoxyphenyl)-4-oxo-4H-chromen-5-yl 5-(1,2-dithiolan-3-yl)pentanoat;
- 7-Acetoxy-2-(3,4-diacetoxyphenyl)-4-oxo-4H-chromen-5-yl-2,5,7,8-tetramethyl-2-(4,8,12-trimethyltridecyl)-3,4-dihydro-2H-chromen-6-yl-succinat;
- 2-Methoxy-4-(7-(3-methoxy-4-(prop-1-en-2-yl oxy)phenyl)-3,5-dioxoheptyl)phenyl 5-(1,2-dithiolan-3-yl)pentanoat; und
- 2-Methoxy-4-(7-(3-methoxy-4-(prop-1-en-2-yloxy)phenyl)-3,5-dioxoheptyl)phenyl 2,5,7,8-tetramethyl-2-(4,8, 12-trimethyltridecyl)-3,4-dihydro-2H-chromen-6-yl-succinat.

13. Verwendung nach Anspruch 12, bei der der Bio-Precursor ausgewählt ist aus (E)-4-(3,5-Diacetoxystyryl)phenyl-5-(1,2-dithiolan-3-yl) pentanoat und (E)-4-(3,5-Diacetoxystyryl)phenyl-2,5,7,8-tetramethyl-2-(4,8,12-trimethyllridecyl)-3,4-dihydro-2H-chromen-6-yl-succinat.

14. Bio-Precursor, der der Struktur entspricht:
[A]ₙ-PP-[B]ₘ
in der:
- PP einen Rest eines Polyphenols darstellt, bei dem jede Hydroxylfunktion durch eine Gruppe A oder eine Gruppe B geschützt ist;
- A eine substituierte oder nichtsubstituierte, gesättigte oder nichtgesättigte Alkylkette ist, die 1 bis 20, vorzugsweise 1 bis 4, Kohlenstoffatome umfasst, die an das Polyphenol gebunden ist durch:
- eine Ester-Carboxylfunktion an einer Hydroxylfunktion des Polyphenols; oder
- über einen Spacer A', bei dem A durch eine Ester-Carboxylfunktion an A' gebunden ist und A' an das Phenol durch eine Ester-Carboxylfunktion an eine Hydroxylfunktion des Phenols gebunden ist;
- n eine ganze Zahl größer oder gleich 1, insbesondere 1, 2, 3, 4 oder 5 darstellt;
- B ein Precursor eines biologisch aktiven Moleküls ist, das an das Polyphenol durch:
- eine Ester-Carboxylfunktion an eine Hydroxylfunktion des Phenols gebunden ist; oder
- über einen Spacer B', bei dem B durch eine Ester-Carboxylfunktion an B' gebunden ist, und B' an das Polyphenol durch eine Ester-Carboxylfunktion an eine Hydroxylfunktion des Polyphenols gebunden ist;
wobei das biologisch aktive Molekül ausgewählt ist aus: einem kosmetisch aktiven Molekül, einem pharmazeutisch aktiven Molekül, einem dermatologisch aktiven Molekül,
wobei das kosmetisch aktive Molekül, das pharmazeutisch aktive Molekül oder das dermatologisch aktive Molekül ausgewählt ist aus: Polyphenol, Lipoidsäure, Vitaminen A, B, D, E, F, Retinsäuren,
- m eine ganze Zahl größer oder gleich 1, insbesondere 1 oder 2, darstellt,
die Gruppe A unterschiedlich zur Gruppe B ist.

15. Bio-Precursor nach Anspruch 14, **dadurch gekennzeichnet, dass** das Polyphenol PP von einer Art ist, die mindestens 2 Phenolkerne aufweist.

16. Bio-precursor nach Anspruch 14, **dadurch gekennzeichnet, dass** das Polyphenol PP folgender Formel entspricht: 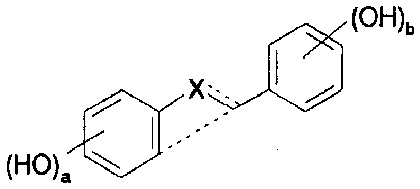 in der:
• a 1, 2, 3, 4 oder 5 ist
• b 1, 2 ,3 ,4 oder 5 ist
• X N, S, O CH, CH₂, CO, NH ist,
• ---- eine doppelte oder eine einfache Bindung darstellt
• ----eine gegebenenfalls vorhandene Kette darstellt, die einen Zyklus mit fünf oder sechs Kettengliedern bildet, die X einschließen und ein oder mehrere Doppelbindungen, gegebenenfalls ein oder mehrere Substituenten OH und/oder ein oder mehrere Heteroatome aufweisen, die ausgewählt sind aus N, S, O, die in dem Zyklus angeordnet sind und/oder als Substituent.

17. Bio-Precursor nach Anspruch 14, **dadurch gekennzeichnet, dass** das Polyphenol PP folgender Formel entspricht: 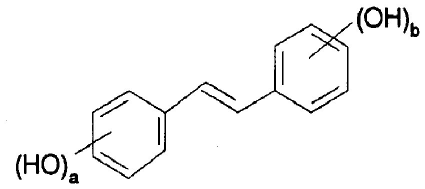 in dem a 1, 2, 3, 4 oder 5 und b 1, 2, 3, 4 oder 5 ist.

18. Bio-Precursor nach Anspruch 17, **dadurch gekennzeichnet, dass** a gleich 2 und b gleich 1 ist.

19. Bio-Precursor nach Anspruch 14, **dadurch gekennzeichnet, dass** das Polyphenol PP der folgenden Formel entspricht: 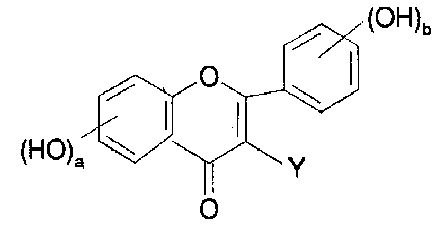 in der Y H oder OH ist, a 1, 2, 3 oder 4 und b 1, 2, 3, 4 oder 5 ist.

20. Bio-Precursor nach Anspruch 19, **dadurch gekennzeichnet, dass** a 2 und b 2 ist.

21. Bio-Precursor nach Anspruch 14, **dadurch gekennzeichnet, dass** das Polyphenol PP ausgewählt ist aus: Resveratrol, Luteolin, Quercetin, Hydroquinon, Pyrocatechol, Gallussäure, Hydroxytyrosol, Tetrahydrocurcumin, Silylmarin, Ellagsäure.

22. Bio-Precursor nach einem beliebigen der Ansprüche 14 bis 21, **dadurch gekennzeichnet, dass** A aus einer linearen Carboxylsäure, verzweigt oder zyklisch, herrührt, die ausgewählt ist aus Essigsäure, Propionsäure, Buttersäure, linear oder verzweigt, Capronsäure und Laurinsäure.

23. Bio-Precursor nach einem beliebigen der Ansprüche 14 bis 21, **dadurch gekennzeichnet, dass** A' und B' unabhängig voneinander eine Kohlenwasserstoffkette, vorzugsweise eine aliphatische Kohlenwasserstoffkette sind, die zwei Säurefunktionen oder eine Säurefunktion und eine Hydroxylfunktion aufweist, die 2 bis 13, vorzugsweise 2 bis 5 Kohlenstoffatome umfasst.

24. Bio-Precursor nach Anspruch 23, **dadurch gekennzeichnet, dass** A' und B' unabhängig voneinander ein Bernsteinsäure-, Adipinsäure-, Brassylsäure-, Milchsäure-, Silicylsäure-, 4-Hydroxybenzoesäure-, Ferulasäure, Apfelsäure-, 2-Hydroxybutansäure-, 3-Hydroxybutansäure-, 4-Hydroxybutansäurerest sind.

25. Bio-Precursor nach Anspruch 14, ausgewählt aus:
- (E)-4-(3,5-Diacetoxystyryl)phenyl-5-(1,2-dithiolan-3-yl) pentanoat;
- (E)-4-(3,5-Diacetoxystyryl)phenyl-2,5,7,8-tetramethyl-2-(4,8,12-trimethyltridecyl)-3,4-dihydro-2H-chromen-6-yl-succinat;
- 4-((E)-3,5-Diacetoxystyryl)phenyl-(2Z,4E,6Z,8E)-3,7-dimethyl-9-(2,6,6-trimethylcyclohex-1-enyl)nona-2,4, 6,8-tetraenylsuccinat;
- (2E,4E,6E,8E, 10E,12E)-4-((E)-3,5-Diacetoxystyryl)phenyl docosa-2,4,6,8,10,12-hexaenoat;
- (E)-4-(3,5-Diacetoxystyryl)phenyl 4-hydroxy-3-methoxybenzoat;
- (E)-4-(3,5-Diacetoxystyryl)phenyl 3,4-diacetoxybenzoat;
- (E)-4-((E)-3,5-Diacetoxystyryl) phenyl 3-(4-hydroxy-3-Methoxyphenyl)acrylat;
- (E)-4-((E)-3,5-Diacetoxystyryl) phenyl 3-(4-acetoxy-3-methoxyphenyl)acrylat;
- (E)-4-((E)-3,5-Diacetoxystyryl)phenyl 3-(4-methoxyphenyl)acrylat;
- (E)-4-(3,5-Diacetoxystyryl)phenyl 2-acetoxybenzoat;
- 7-Acetoxy-2-(3,4-diacetoxyphenyl)-4-oxo-4H-chromen-5-yl 5-(1,2-dithiolan-3-yl)pentanoat;
- 7-Acetoxy-2-(3,4-diacetoxyphenyl)-4-oxo-4H-chromen-5-yl-2,5,7,8-tetramethyl-2-(4,8,12-trimethyltridecyl)-3,4-dihydro-2H-chromen-6-yl-succinat;
- 2-Methoxy-4-(7-(3-methoxy-4-(prop-1-en-2-yl oxy)phenyl)-3,5-dioxoheptyl)phenyl 5-(1,2-dithiolan-3-yl)pentanoat; und
- 2-Methoxy-4-(7-(3-methoxy-4-(prop-1-en-2-yloxy)phenyl)-3,5-dioxoheptyl)phenyl 2,5,7,8-tetramethyl-2-(4,8, 12-trimethyltridecyl)-3,4-dihydro-2H-chromen-6-yl-succinat.

26. Bio-Precursor nach Anspruch 25, ausgewählt aus (E)-4-(3,5-Diacetoxystyryl)phenyl-5-(1,2-dithiolan-3-yl)-pentanoat und (E)-4-(3,5-Diacetoxystyryl)phenyl-2,5,7,8-tetramethyl-2-(4,8,12-trimethyllridecyl)-3,4-dihydro-2H-chromen-6-yl-succinat.

27. Zusammensetzung mindestens eines Bio-Precursors nach einem beliebigen der Ansprüche 14 bis 26 einen Precursor B eines dermatologisch oder kosmetisch aktiven Moleküls in einer topischen Zubereitung, die für eine dermatologische oder kosmetische Anwendung annehmbar ist, umfassend.

28. Zusammensetzung mindestens eines Bio-Precursors nach einem beliebigen der Ansprüche 14 bis 26, einen Precursor B eines therapeutischen Moleküls in einer topischen Zubereitung, die für eine therapeutische Anwendung annehmbar ist, umfassend.

29. Verfahren zur kosmetischen Behandlung, die Anwendung einer kosmetischen Zusammensetzung nach Anspruch 27 auf der Haut umfassend.

30. Verfahren zur Herstellung eines Bio-Precursors nach einem beliebigen der Ansprüche 14 bis 26, mindestens die folgenden Schritte umfassend:
a) Schutz des Polyphenols durch Veresterung durch eine Verbindung A - Z, wobei Z eine Funktion ist, die geeignet ist, mit einer Funktion OH des Polyphenols zu reagieren, um die Esterbindung zwischen dem Polyphenol und A zu erzeugen,
b) Selektives Entschützen derart, dass eine oder mehrere freie OH-Funktionen des Polyphenols erhalten werden, und
c) Koppeln des nach dem Schritt b) erhaltenen Zwischenergebnisses mit dem biologisch aktiven Molekül B, das vorher aktiviert wurde.

## Claims

1. Use of a bioprecursor which has the structure:
[A]ₙ - PP - [B]ₘ
in which:
- PP represents a polyphenol radical in which each hydroxyl function is protected by a group A or a group B;
- A is a substituted or unsubstituted, saturated or unsaturated alkyl chain comprising from 1 to 20 carbon atoms, preferably from 1 to 4, which is bound to the polyphenol by:
- a carboxylic ester function on a hydroxyl function of the said polyphenol; or
- by means of an A' spacer, in which A is bound to A' by a carboxylic ester function, and A' is bound to the polyphenol by a carboxylic ester function on a hydroxyl function of the said polyphenol;
- n represents an integer greater than or equal to 1, in particular 1, 2, 3, 4 or 5;
- B is a precursor of a biologically active molecule which is bound to the polyphenol by:
- a carboxylic ester function on a hydroxyl function of the said polyphenol; or
- by means of a B' spacer, in which B is bound to B' by a carboxylic ester function, and B' is bound to the polyphenol by a carboxylic ester function on a hydroxyl function of the said polyphenol;
said biologically active molecule being chosen among a cosmetically active molecule, a pharmaceutically active molecule, a dermatologically active molecule,
said cosmetically active, pharmaceutically active or dermatologically active molecule being chosen among polyphenol, lipoic acid, vitamins A, B, D, E, F, retinoic acids,
- m represents an integer greater than or equal to 1, in particular 1 or 2,
- the A group being different from the B group
In a topical formulation, for the preparation of a therapeutic, dermatological or cosmetic composition.

2. Use according to Claim 1, **characterised in that** the polyphenol is of the type comprising at least 2 phenol nuclei.

3. Use according to Claim 1, **characterised in that** the polyphenol complies with the following formula: 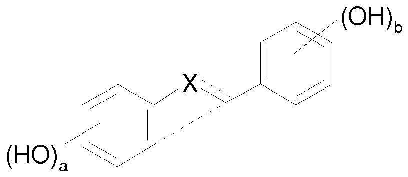 in which:
• a is 1, 2, 3, 4 or 5;
• b is 1, 2, 3, 4 or 5;
• X is N, S, O, CH, CH₂, CO, NH ;
• ----represents a double or a single bond;
• ---- represents a chain which may be present, forming a ring with 5 or 6 links, including X and having one or more double bonds, possibly one or more OH substituents and/or one or more heteroatoms chosen from amongst N, S, O, which are situated in the ring and/or substitute therefor.

4. Use according to Claim 1, **characterised in that** the polyphenol complies with the following formula: 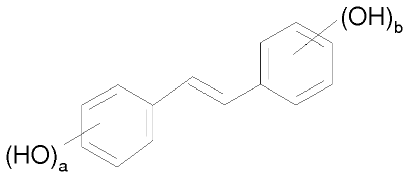 in which a is 1, 2, 3, 4 or 5 and b is 1, 2, 3, 4 or 5.

5. Use according to Claim 4, **characterised in that** a is 2 and b is 1.

6. Use according to Claim 1, **characterised in that** the polyphenol complies with the following formula: 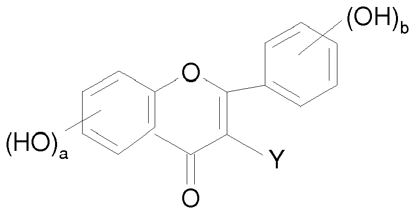 in which Y is H or OH, a is 1, 2, 3, or 4 and b is 1, 2, 3, 4 or 5.

7. Use according to Claim 6, **characterised in that** a is 2 and b is 2.

8. Use according to Claim 1, **characterised in that** the polyphenol is chosen from amongst: resveratrol, luteolin, quercetin, hydroquinone, pyrocatechol, gallic acid, hydroxytyrosol, tetrahydrocurcumin, silymarin, ellagic acid.

9. Use according to any one of the preceding claims, **characterised in that** the group A of the bioprecursor is derived from a linear, branched or cyclic carboxylic acid, chosen from amongst ethanoic acid, propanoic acid, linear or branched butanoic acid, caproic acid and lauric acid.

10. Use according to claim 1, **characterised in that** the groups A' and B' of the bioprecursor are, independently of one another, a preferably aliphatic hydrocarbon chain comprising two acid functions or one acid function and one hydroxyl function comprising from 2 to 13, preferably 2 to 5, carbon atoms.

11. Use according to Claim 10, **characterised in that** A' and B' are independently of one another a succinic acid, adipic acid, brassylic acid, lactic acid, salicylic acid, 4-hydroxybenzoic acid, ferulic acid, tartaric acid, 2-hydroxybutanoic acid, 3-hydroxybutanoic acid or 4-hydroxybutanoic acid radical.

12. Use according to Claim 1, in which the bioprecursor is chosen from amongst:
- (E)-4-(3,5-diacetoxystyryl)phenyl-5-(1,2-dithiolan-3-yl) pentanoate;
- (E)-4-(3,5-diacetoxystyryl)phenyl-2,5,7,8-tetramethyl-2-(4,8,12-trimethyltridecyl)-3,4-dihydro-2*H*-chromen-6-yl succinate;
- 4-((E)-3,5-diacetoxystyryl)phenyl-(2Z,4E,6Z,8E)-3,7-dimethyl-9-(2,6,6-trimethylcyclohex-1-enyl)nona-2,4,6,8-tetraenyl succinate;
- (2E,4E,6E,8E,10E,12E)-4-((E)-3,5-diacetoxystyryl)phenyl docosa-2,4,6,8,10,12-hexaenoate;
- (E)-4-(3,5-diacetoxystyryl)phenyl 4-hydroxy-3-methoxybenzoate;
- (E)-4-(3,5-diacetoxystyryl)phenyl 3,4-diacetoxybenzoate;
- (E)-4-((E)-3,5-diacetoxystyryl)phenyl 3-(4-hydroxy-3-methoxyphenyl)acrylate;
- (E)-4-((E)-3,5-diacetoxystyryl)phenyl 3-(4-acetoxy-3-methoxyphenyl)acrylate;
- (E)-4-((E)-3,5-diacetoxystyryl)phenyl 3-(4-methoxyphenyl)acrylate;
- (E)-4-(3,5-diacetoxystyryl)phenyl 2-acetoxybenzoate;
- 7-acetoxy-2-(3,4-diacetoxyphenyl)-4-oxo-4H-chromen-5-yl 5-(1,2-dithiolan-3-yl) pentanoate;
- 7-acetoxy-2-(3,4-diacetoxyphenyl)-4-oxo-4H-chromen-5-yl 2,5,7,8-tetramethyl-2-(4,8,12-trimethyltridecyl)-3,4-dihydro-2H-chromen-6-yl succinate;
- 2-methoxy-4-(7-(3-methoxy-4-(prop-1-en-2-yloxy)phenyl)-3,5-dioxoheptyl)phenyl 5-(1,2-dithiolan-3-yl) pentanoate; and
- 2-methoxy-4-(7-(3-methoxy-4-(prop-1-en-2-yloxy)phenyl)-3,5-dioxoheptyl)phenyl 2,5,7,8-tetramethyl-2-(4,8,12-trimethyltridecyl)-3,4-dihydro-2H-chromen-6-yl succinate.

13. Use according to Claim 12, in which the bioprecursor is chosen from amongst (E)-4-(3,5-diacetoxystyryl)phenyl-5-(1,2-dithiolan-3-yl) pentanoate and (E)-4-(3,5-diacetoxystyryl)phenyl-2,5,7,8-tetramethyl-2-(4,8,12-trimethyltridecyl)-3,4-dihydro-2*H-*chromen-6-yl succinate.

14. Bioprecursor having the structure:
[A]ₙ - PP - [B]ₘ
in which:
- PP represents a polyphenol radical in which each hydroxyl function is protected by a group A or a group B;
- A is a substituted or unsubstituted, saturated or unsaturated alkyl chain comprising from 1 to 20 carbon atoms, preferably from 1 to 4, which is bound to the polyphenol by:
- a carboxylic ester function on a hydroxyl function of the said polyphenol; or
- by means of an A' spacer, in which A is bound to A' by a carboxylic ester function, and A' is bound to the polyphenol by a carboxylic ester function on a hydroxyl function of the said polyphenol;
- n represents an integer greater than or equal to 1, in particular 1, 2, 3, 4 or 5;
- B is a precursor of a biologically active molecule which is bound to the polyphenol by:
- a carboxylic ester function on a hydroxyl function of the said polyphenol; or
- by means of a B' spacer, in which B is bound to B' by a carboxylic ester function, and B' is bound to the polyphenol by a carboxylic ester function on a hydroxyl function of the said polyphenol;
said biologically active molecule being chosen among a cosmetically active molecule, a pharmaceutically active molecule, a dermatologically active molecule,
said cosmetically active, pharmaceutically active or dermatologically active molecule being chosen among polyphenol, lipoic acid, vitamins A, B, D, E, F, retinoic acids,
- m represents an integer greater than or equal to 1, in particular 1 or 2 the A group being different from the B group.

15. Bioprecursor according to Claim 14, **characterised in that** the polyphenol is of the type comprising at least 2 phenol nuclei.

16. Bioprecursor according to Claim 14, **characterised in that** the polyphenol complies with the following formula: 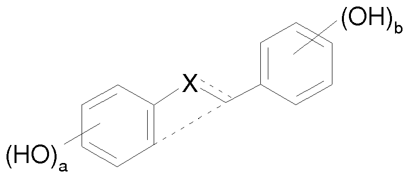 **in that**:
• a is 1, 2, 3, 4 or 5;
• b is 1, 2, 3, 4 or 5;
• X is N, S, O, CH, CH₂, CO, NH;
• ----represents a double or a single bond;
• ---- represents a chain which may be present, forming a ring with 5 or 6 links, including X and having one or more double bonds, possibly one or more OH substituents and/or one or more heteroatoms chosen from amongst N, S, O, which are situated in the ring and/or substitute therefor.

17. Bioprecursor according to Claim 14, **characterised in that** the polyphenol complies with the following formula: 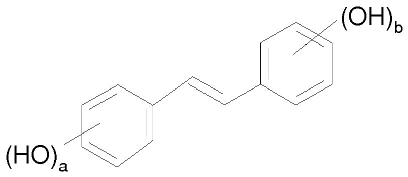 in which a is 1, 2, 3, 4 or 5 and b is 1, 2, 3, 4 or 5.

18. Bioprecursor according to Claim 17, **characterised in that** a is 2 and b is 1.

19. Bioprecursor according to Claim 14, **characterised in that** the polyphenol complies with the following formula: 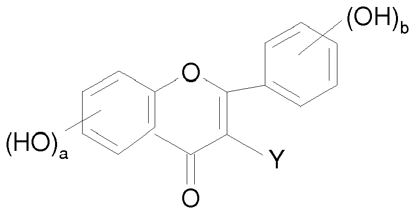 in which Y is H or OH, a is 1, 2, 3 or 4 and b is 1, 2, 3, 4 or 5.

20. Bioprecursor according to Claim 19, **characterised in that** a is 2 and b is 2.

21. Bioprecursor according to Claim 14, **characterised in that** the polyphenol is chosen from amongst: resveratrol, luteolin, quercetin, hydroquinone, pyrocatechol, gallic acid, hydroxytyrosol, tetrahydrocurcumin, silymarin, ellagic acid.

22. Bioprecursor according to any one of Claims 14 to 21, **characterised in that** A is derived from a linear, branched or cyclic carboxylic acid, chosen from amongst ethanoic acid, propanoic acid, linear or branched butanoic acid, caproic acid and lauric acid.

23. Bioprecursor according to any one of Claims 14 to 21, **characterised in that** A' and B' are, independently of one another, a preferably aliphatic hydrocarbon chain comprising two acid functions or one acid function and one hydroxyl function comprising from 2 to 13, preferably 2 to 5 carbon atoms.

24. Bioprecursor according to Claim 23, **characterised in that** A' and B' are independently of one another a succinic acid, adipic acid, brassylic acid, lactic acid, salicylic acid, 4-hydroxybenzoic acid, ferulic acid, tartaric acid, 2-hydroxybutanoic acid, 3-hydroxybutanoic acid or 4-hydroxybutanoic acid radical.

25. Bioprecursor according to Claim 14, chosen from amongst:
- (E)-4-(3,5-diacetoxystyryl)phenyl-5-(1,2-dithiolan-3-yl) pentanoate;
- (E)-4-(3,5-diacetoxystyryl)phenyl-2,5,7,8-tetramethyl-2-(4,8,12-trimethyltridecyl)-3,4-dihydro-2*H*-chromen-6-yl succinate;
- 4-((E)-3,5-diacetoxystyryl)phenyl-(2Z,4E,6Z,8E)-3,7-dimethyl-9-(2,6,6-trimethylcyclohex-1-enyl)nona-2,4,6,8-tetraenyl succinate;
- (2E,4E,6E,8E,10E,12E)-4-((E)-3,5-diacetoxystyryl)phenyl docosa-2,4,6,8,10,12-hexaenoate;
- (E)-4-(3,5-diacetoxystyryl)phenyl 4-hydroxy-3-methoxybenzoate;
- (E)-4-(3,5-diacetoxystyryl)phenyl 3,4-diacetoxybenzoate;
- (E)-4-((E)-3,5-diacetoxystyryl)phenyl 3-(4-hydroxy-3-methoxyphenyl)acrylate;
- (E)-4-((E)-3,5-diacetoxystyryl)phenyl 3-(4-acetoxy-3-methoxyphenyl)acrylate;
- (E)-4-((E)-3,5-diacetoxystyryl)phenyl 3-(4-methoxyphenyl)acrylate;
- (E)-4-(3,5-diacetoxystyryl)phenyl 2-acetoxybenzoate;
- 7-acetoxy-2-(3,4-diacetoxyphenyl)-4-oxo-4H-chromen-5-yl 5-(1,2-dithiolan-3-yl) pentanoate;
- 7-acetoxy-2-(3,4-diacetoxyphenyl)-4-oxo-4H-chromen-5-yl 2,5,7,8-tetramethyl-2-(4,8,12-trimethyltridecyl)-3,4-dihydro-2H-chromen-6-yl succinate;
- 2-methoxy-4-(7-(3-methoxy-4-(prop-1-en-2-yloxy)phenyl)-3,5-dioxoheptyl)phenyl 5-(1,2-dithiolan-3-yl) pentanoate; and
- 2-methoxy-4-(7-(3-methoxy-4-(prop-1-en-2-yloxy)phenyl)-3,5-dioxoheptyl)phenyl 2,5,7,8-tetramethyl-2-(4, 8,12-trimethyltridecyl)-3,4-dihydro-2H-chromen-6-yl succinate.

26. Bioprecursor according to Claim 25, in which the bioprecursor is chosen from amongst (E)-4-(3,5-diacetoxystyryl)phenyl-5-(1,2-dithiolan-3-yl) pentanoate and (E)-4-(3,5-diacetoxystyryl)phenyl-2,5,7,8-tetramethyl-2-(4,8,12-trimethyltridecyl)-3,4-dihydro-2H-chromen-6-yl succinate.

27. Composition comprising at least one bioprecursor according to any one of Claims 14 to 26, comprising a precursor B of a dermatologically or cosmetically active molecule, in a topical formulation which is acceptable for a dermatological or cosmetic application.

28. Composition comprising at least one bioprecursor according to any one of Claims 14 to 26, comprising a precursor B of a therapeutic molecule, in a topical formulation which is acceptable for a therapeutic application.

29. Process for cosmetic or dermatological treatment, comprising the application on the skin of a composition according to Claim 27.

30. Process for manufacture of a bioprecursor according to any one of Claims 14 to 26, comprising at least the following steps:
a) protection of the polyphenol by peresterification by a compound A-Z, Z being a function capable of reacting with an OH function of the polyphenol in order to generate the ester bond between the polyphenol and A,
b) selective deprotection in such a way as to obtain one or more free OH functions of the polyphenol, and
c) coupling of the intermediate obtained after the step b) with the biologically active B molecule previously activated.
